(19) Europäisches Patentamt
European Patent Office
Office européen des brevets

(11) EP 1 268 421 B1

(12) **EUROPEAN PATENT SPECIFICATION**

(45) Date of publication and mention of the grant of the patent:
**06.05.2009 Bulletin 2009/19**

(21) Application number: **01920898.2**

(22) Date of filing: **29.03.2001**

(51) Int Cl.:
***C07D 209/12*** (2006.01) ***C07D 403/12*** (2006.01)
***C07D 401/12*** (2006.01) ***A61K 31/404*** (2006.01)
***A61P 25/28*** (2006.01)

(86) International application number:
**PCT/US2001/010320**

(87) International publication number:
**WO 2001/074773 (11.10.2001 Gazette 2001/41)**

(54) **PHENYL-SUBSTITUTED INDOLES AS HISTAMINE H3-RECEPTOR ANTAGONISTS**

PHENYL-SUBSTITUIERTE INDOLE ALS H3 HISTAMINREZEPTORANTAGONISTEN

INDOLES SUBSTITUES PAR UN PHENYLE COMME ANTAGONISTES DU RECEPTEUR HISTAMINIQUE H3

(84) Designated Contracting States:
**AT BE CH CY DE DK ES FI FR GB GR IE IT LI LU MC NL PT SE TR**

(30) Priority: **31.03.2000 US 194071 P**
**28.02.2001 US 272287 P**

(43) Date of publication of application:
**02.01.2003 Bulletin 2003/01**

(73) Proprietor: **Ortho-McNeil Pharmaceutical, Inc.**
**Raritan, NJ 08869-0602 (US)**

(72) Inventors:
- **BREITENBUCHER, J., Guy**
**Escondido, CA 92026 (US)**
- **CHAI, Wenying**
**San Diego, CA 92126 (US)**

(74) Representative: **Fisher, Adrian John et al**
**CARPMAELS & RANSFORD**
**43-45 Bloomsbury Square**
**London WC1A 2RA (GB)**

(56) References cited:
**WO-A-98/06703**
**WO-A-98/48797**
**WO-A-99/33822**
**US-A- 338 106**
**US-A- 5 385 912**
**US-A- 5 681 954**

- **DATABASE CA [Online] CHEMICAL ABSTRACTS SERVICE, COLUMBUS, OHIO, US; AGARWAL, SHIV K. ET AL: "Synthesis and pharmacological activities of 1-(2,4-disubstituted phenoxy)-3-[N1-(N4-arylpiperazinyl)]propan es and 1-(4-chlorobenzoyl)-3-substituted 6-methoxy-2-{4-[3-N1-(N4- phenylpiperazinyl)propoxy]phenyl} indoles" retrieved from STN Database accession no. 115:49618 XP002182674 & INDIAN J. CHEM., SECT. B (1991), 30B(4), 413-16 ,**
- **DATABASE CA [Online] CHEMICAL ABSTRACTS SERVICE, COLUMBUS, OHIO, US; JOSHI, KRISHNA C. ET AL: "Preparation of new fluorine containing 2-phenylindole derivatives as antifertility agents" retrieved from STN Database accession no. 105:190834 XP002182675 & J. INDIAN CHEM. SOC. (1985), 62(5), 388-90 ,**
- **GANELLIN C R ET AL: "Synthesis of potent non-imidazole histamine H3-receptor antagonists" ARCHIV DER PHARMAZIE, VCH VERLAGSGESELLSCHAFT MBH, WEINHEIM, DE, 1998, pages 395-404, XP002123596 ISSN: 0365-6233 cited in the application**
- **TAKEUCHI, K. ET AL: "1,2-disubstituted indole, azaindole and benzimidazole derivatives possessing amine moiety: a novel series of thrombin inhibitors" BIOORG. MED. CHEM. LETT. (2000), 10(20), 2347-2351 , XP002182673**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The invention relates to pharmaceutically-active fused heterobicyclic compounds and their use in the manufacture of medicaments to treat or prevent disorders and conditions, such as those mediated by the histamine $H_3$ receptor.

### Background

The histamine $H_3$ receptor Is located as a presynaptic autoreceptor in the central nervous system and as a presynaptic heteroreceptor on serotonergic, noradrenergic, dopaminergic, and cholinergic neurons. The histamine $H_3$ receptor is also located peripherally in tissues such as vascular smooth muscle cells.

Proposed uses of histamine $H_3$ antagonists include the treatment or prevention of dementia, Alzheimer's disease (Panula et al. Abstr. Society Neuroacience, 1995, 21:1977), epilepsy (Yokoyama et al. Eur. J. Pharmacol., 1993, 234: 129), sleep/wake disorders (Lin et al., Br. Res., 1990, 523, 325; Monti et al., Eur. J. Pharmacol., 1991, 205, 283) including narcolepsy, insomnia, and jet lag, eating disorders (Machidori et al. Brain Research, 1992. 590:180), motion sickness, vertigo, attention deficit hyperactivity disorder, learning and memory disorders (Barnes et al. Abstr. Society Neuroscience, 1993, 19:1813), schizophrenia (Schlicker et al, Naunyn Schmiedeberg's Arch. Pharmacol., 1996, 353:326), and sequelae associated with post-ischemic reperfusion and hypertension. (Imamura et al., J. Pharmacol. Expt. Ther., 1994, 271, 1259). $H_s$ antagonists are also useful to treat or prevent neurogenic inflammation such as migraine (McLeod et a/., Abstr. Society Neuroscience, 1996, 22, 2010), asthma (Ichinose et al., Eur. J. Pharmacol., 989, 174, 49), obesity, allergic rhinitis, substance abuse, bipolar disorders, manic disorders, and depression. Histamine $H_3$ antagonists alone or In combination with a histamine H, antagonist are believed to be useful In the treatment of upper airway allergic response or allergic rhinitis (US Patent Nos. 5217986, 5352707, and 5869479).

As noted, the prior art related to histamine $H_3$ ligands was comprehensively reviewed recently *("*The Histamine H9 Receptor-A Target for New Drugs", Leurs, R12 and Timmerman, H., (Editors), Elsevier, 1998). Within this reference the medicinal chemistry of histamine $H_3$ agonists and antagonists was reviewed (see Krause et al. and Phillips et al., respectively). Thus the importance of an imidazole moiety containing only a single substitution in the 4 position was noted together with the deleterious effects of additional substitution on activity. Particularly methylation of the Imidazole ring at any of the remaining unsubstituted positions was reported to strongly decrease activity.

More recently several publications have described histamine $H_3$ ligands that do not contain an imidazole moiety. Examples include Ganellin et al Arch. Pharm. (Weinhelm, Ger.) 1998, 331, 395; Walczynski et al Arch. Pharm. (Weinhelm, Ger.) 1999, 332, 389; Walczynski et al Fannaco 1999, 684; Linney et al J. Med. Chem. 2000, 2362; U.S. Patent 5,352,707; PCT Application WO99/42458, published August 26, 1999; and European Patent Application 0978512, published on February 9, 2000.

### Summary of the Invention

The invention features phenyl-substituted indole and indazole compounds, and uses of them. One aspect of the invention provides compounds of the following formula (I)(B):

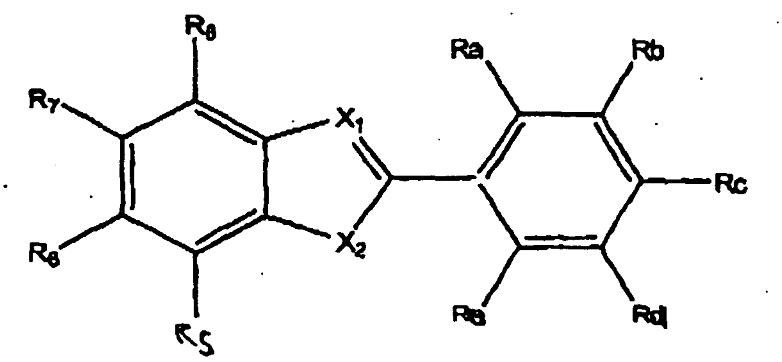

wherein $X_1$ is $CR_1$, wherein $R_1$ is H, halo, cyano, amino, or nitro; and $X_2$ is $NR_3$;
$R_3$ is -W'Z':
W' is (C=O) or $SO_2$;

Z' is C $_{1-6}$ alkyl, C $_{1-6}$ alkoxy, C $_{3-8}$ cycloalkyl, phenyl, or C $_{2-6}$ heterocyclic radical, optionally including In the ring up to 3 additional heteroatoms or moieties independently selected from O, N, NH, S, SO, and $SO_2$; or Z' is $NR_{13}R_{14}$ where each of $R_{13}$ and $R_{14}$ is independently selected from C $_{1-6}$ alkyl, C $_{2-6}$ alkenyl, phenyl, benzyl, C $_{3-8}$ cycloalkyl, and C $_{2-6}$ heterocyclic radical;

each of $R_5$, $R_6$, $R_7$ and $R_8$ is independently H, C $_{1-6}$ alkyl, C $_{1-6}$ alkoxy, halo, nitro, or amino;

one of $R_a$, $R_b$, $R_c$, $R_d$, and $R_e$ is WZ and the others are independently selected from H, C $_{1-6}$ alkyl, C $_{1-6}$ alkoxy, halo, nitro, and amino;

W is -O-, or O-$R_9$, wherein $R_9$ is C $_{1-6}$ alkylene, C $_{2-6}$ alkynylene, C $_{2-6}$ alkenylene, phenylene, or $C_{2-6}$ heterocyclic bivalent radical;

Z is C $_{2-6}$ heterocyclic radical with at least one basic nitrogen atom In the ring, optionally including in the ring up to 3 additional heteroatoms or moieties Independently selected from O, C=O, N, NH, NG, S, SO, and $SO_2$, wherein G is $R_{15}$, $COR_{15}$, $COOR_{15}$, $SO_2R_{15}$, $SO_2N$, $CSR_{15}$; or Z is $NR_{11}R_{12}$ where each of $R_{11}$ and $R_{12}$ is independently selected from H, C $_{1-6}$ alkyl, phenyl, benzyl, C $_{3-8}$ cycloalkyl, and C $_{2-5}$ heterocyclic radical; or $NR_{11}R_{12}$ taken together is a C $_{6-8}$ cycloalkylimino radical; and $R_{15}$ is C $_{1-6}$ alkyl. C $_{2-6}$ alkynyl, C $_{2-6}$ alkenyl, C $_{3-7}$ cycloalkyl, and C $_{4-7}$ cycloalkenyl;

each of the above hydrocarbyl or heterocyclic groups being optionally substituted with between 1 and 3 substituents selected from C $_{1-3}$ alkyl, C $_{1-3}$ alkoxy, halo, hydroxy, phenyl, and phenyl(C $_{1-3}$ alkyl); and wherein each of the above heterocyclic groups may be attached to the rest of the molecule by a carbon atom or a heteroatom;

or a pharmaceutically acceptable said, or hydrate thereof.

According to another aspect of the invention, the disclosed compounds are useful in the manufacture of medicaments for the treatment and/or prevention of diseases and conditions mediated by the histamine 3 ($H_3$) receptor.

Disclosed herein are methods of making the disclosed compounds.

Additional features of the invention are disclosed in the following description and examples, and in the appended claims.

## Detailed Description

Disclosed herein are pharmaceutically active phenyl-substituted indoles and indazoles and methods of making and using them. The description is organized as follows:

A. Terms
B. Compounds
C. Synthetic Methods
D. Uses (including dosages, formulations, and related compounds)
E. Synthetic Examples
F. Biological Examples
G. Other Embodiments
H. Claims

### A. Terms

The following terms are defined below and by their usage throughout this disclosure.

"Alkyl" includes straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl does not include cycloalkyl.

"Alkenyl" includes straight chain and branched hydrocarbon radicals as above with at least one carbon-carbon double bond ($sp^2$). Alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on. Hydrocarbon radicals having a mixture of double bonds and triple bonds, such as 2-penten-4-ynyl, are grouped as alkynyls herein. Alkenyl does not include cycloalkenyl.

"Alkynyl" include straight chain and branched hydrocarbon radicals as above with at least one carbon-carbon triple bond (sp). Alkynyls include ethynyl, propynyls, butynyls, and pentynyls. Hydrocarbon radicals having a mixture of double bonds and triple bonds, such as 2-penten-4-ynyl, are grouped as alkynyls herein. Alkynyl does not include cycloalkynyl.

"Alkoxy" includes a straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with,

respectively, NH (or NR), S, and $SO_2$.

"Aryl" includes phenyl, naphthyl, biphenylyl, and so on.

"Cycloalkyl" includes cyclopropyl, cyclobutyl, cyclopentyl, cyclohexyl, cycloheptyl, cyclooctyl, and so on.

"Cycloalkenyl" includes cyclobutenyl, cyclobutadienyl, cyclopentenyl, cyclopentadienyl, cyclohexenyl, cyclohexadienyl, cyclohexatrienyl (phenyl), cycloheptenyl, and so on. "Cycloalkynyl" includes the analogous rings with one or more triple bonds.

"Heterocyclic radicals" include aromatic and nonaromatic rings having carbon atoms and at least one heteroatom (O, S, N) or heteroatom moiety ($SO_2$, CO, CONH, COO) in the ring. Unless otherwise indicated, a heterocyclic radical may have a valence connecting it to the rest of the molecule through a carbon atom, such as 3-furyl or 2-imidazolyl, or through a heteroatom, such as N-piperidyl or 1-pyrazolyl. Examples of heterocyclic radicals include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imdazolidinyl, imidazolinyl, pyrazolidinyl, pyrazolinyl, piperidyl, piperazinyl, indolinyl, and morpholinyl. For example, preferred heterocyclic radicals for Z include morpholinyl, piperazinyl, pyrazinyl, pyrrolidinyl, pyridyl, cyclohexylimino, cycloheptylimino,and more preferably, piperidyl.

"halo" includes fluoro, chloro, bromo, and iodo, and preferably fluoro or chloro.

"patient" or "subject" includes mammals such as humans and animals (dogs, cats, horses, rats, rabbits, mice, non-human primates) in need of observation, experiment, treatment or prevention in connection with the relevant disease or condition. Preferably, the patient is a human.

"composition" includes a product comprising the specified ingredients in the specified amounts as well as any product which results directly or indirectly from combinations of the specified ingredients in the specified amounts.

Concerning the various radicals in this disclosure and in the claims, a general remark may be made concerning valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent). An example of a bivalent radical linking two parts of the molecule is W in formula (I)(B) which links Z with the phenyl group (-Ph-WZ). Subject to the claims, W can be an alkyl (strictly, alkylene) group (-Ph-$CH_2CH_2CH_2$-Z), an aminoalkyl group (-Ph-NH-$CH_2CH_2CH_2$-Z), an alkoxy group ( -Ph-O-$CH_2CH_2CH_2$-Z), an "oxs" (-Ph-O-Z), and so on.

B. Compounds

One aspect of the invention features compounds of formula (I)B as described in the Summary section above. The invention encompasses the described compounds and pharmaceutically acceptable salts, and hydrates thereof.

Preferred compounds include those compounds of formula (I)B wherein: (a) $R_3$ is -(C=O)C $_{1-6}$alkyl; (b) $R_3$ is -$SO_2$(C $_{1-3}$ alkyl); (c) $R_3$ is methylsulfonyl; (d) $R_c$ is WZ; (e) $R_b$ or $R_d$ is WZ; (f) W is ethoxy, propoxy, or butoxy; (g) W is -O-; (h) one of $R_b$, $R_c$, and $R_e$ is WZ and the others are independently selected from H, methyl, ethyl, methoxy, ethoxy, amino, nitro, and halo; and $R_a$ and $R_d$ are each independently H or methyl; (l) at least two of the following apply: $R_c$ is WZ; W is propoxy or ethoxy; and Z is N-piperidino, 2-(N-methyl)pyrrolidino, or dimethyl amino; or combinations thereof..

Additional preferred compounds include those wherein (i) Z is piperazino or N-methylpiperazino, and more preferably Z is pyrrolidino, N-methyl-pyrrolidino, pyridyl, thiazoyl, piperidino, or $NR_{11}R_{12}$ where each of $R_{11}$ and $R_{12}$ is Independently selected from H, C $_{1-6}$ alkyl, phenyl, benzyl, C $_{3-6}$ cycloalkyl, and C $_{2-5}$ heterocyclic radical or taken together with the N form a C $_{6-a}$ cycloalkylamino radical; or wherein (j) is combined with (a) through (i) above.

Further preferred compounds include those wherein (k) one of $R_b$, $R_c$, and $R_e$ is WZ and the others are independently selected from H, methyl, ethyl, methoxy, ethoxy, amino, and halo; and $R_a$ and $R_d$ are each Independently H or methyl; W is -O- or C $_{1-3}$ alkoxy; Z is piperazino or N-methylplperaino, and more preferably pyrrolidino. N-methylpyrrolidino, pyridyl, thiazoyl, piperidino, or $NR_{11}R_{12}$ where each of $R_{11}$ and $R_{12}$ is independently selected from H, C $_{1-2}$ alkyl, phenyl, benzyl, C $_{3-6}$ cycloalkyl, and C $_{2-5}$ heterocyclic radical; each of $R_6$ and $R_7$ are each independently H, methyl, methoxy, or ethoxy; and each of $R_5$ and $R_6$ is H. Preferred compounds also include those wherein for example one or more of (a) through (k) is combined with (l) $R_3$ is H or -$SO_2$ (C $_{1-6}$ alkyl); or (m) $R_3$ is $SO_2$ (phenyl) and (C=O)(C $_{1-6}$ alkyl).

Examples of more preferred compounds include: (i) 2-[4-[2-[1-(methyl)-2-pyrrolidinyl]ethoxy]phenyl)-1-(methylsulfonyl) -1H-Indole; 2-[4-[3-Piperidinopropoxy]phenyl)-1-(methylsulfonyl)-1H-indole; and 2-[3-[3-Piperidinopropoxy] phenyl)-1-(mothysulfonyl)-1H-indole; and 1-(methylsulfonyl)-2-(4-(3-(4-methylpiperazino)-propoxy)phenyl)indole.

Other examples of compounds, and methods of making them, are provided In the next section.

C. Synthetic Chemical Methods

Disclosed herein are methods of making the disclosed compounds according to traditional organic synthetic methods as well as matrix or combinatorial synthetic methods. Schemes 1 through 9 describe suggested synthetic routes.

Using these Schemes, the guidelines below, and the examples in section E, a person of skill in the art may develop analogous or similar methods for a given compound.

One skilled in the art will recognize that synthesis of the compounds of the present invention may be effected by purchasing an intermediate or protected intermediate compounds described in any of the schemes disclosed herein. One skilled in the art will further recognize that during any of the processes for preparation of the compounds In the present invention, it may be necessary and/or desirable to protect sensitive or reactive groups on any of the molecules concerned. This may be achieved by means of conventional protecting groups, such as those described In "Protective Groups in Organic Synthesis", John Wiley & Sons, 1991. These protecting groups may be removed at a convenient stage using methods known from the art.

Examples of the described synthetic routes includes Synthetic Example 1 through 17. Compounds analogous to the target compounds of these examples can be, and In many cases, have been, made according to similar routes. The disclosed compounds are useful in basic research and as pharmaceutical agents as described in the next section.

Scheme I

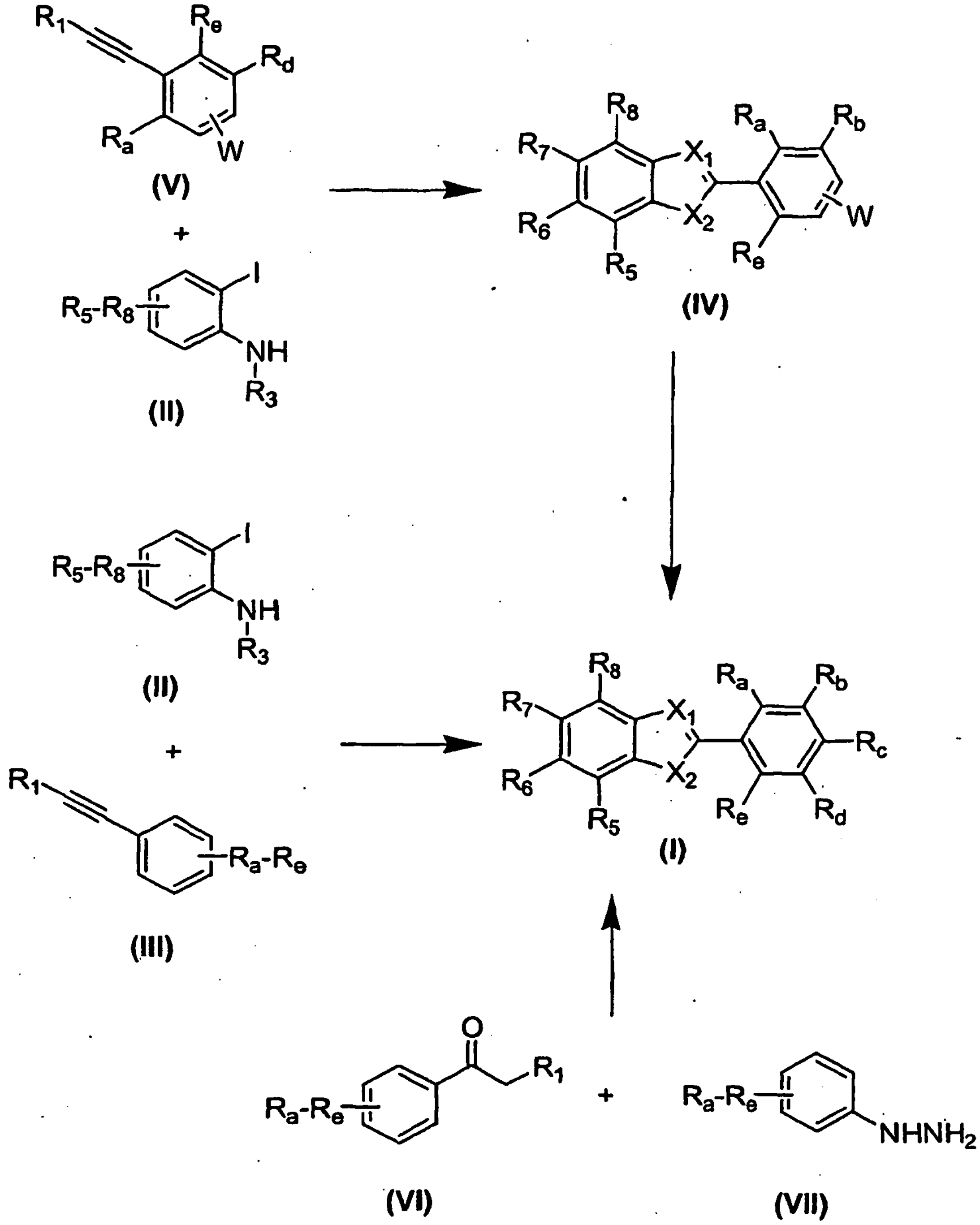

(V) + (II) → (IV) → (I)

(II) + (III) → (I)

(VI) + (VII) → (I)

Generally, a compound of formula (V), a known compound or compound prepared by known methods, is reacted with a compound of formula (II), a known compound or compound prepared by known methods, in the presence of a palladium catalyst such as dichlorobis(triphenylphosphine) palladium, and CuI, or the like, in the presence of a base such as triethylamine, or the like, in a solvent such as DMF, THF, DMA, and the like, to yield the corresponding compound of formula (IV). Compound (IV) is then further reacted, as outlined in Schemes 5-7 below, to form the compound of formula (I). Alternatively a compound of formula (III) can be reacted with a compound of formula (II) using the above described, or similar methods to form a compound of formula (I) directly. In addition compounds of formula (I), in which $X_2$ is NH can be obtained by reacting a compound of formula (VI) with an aromatic hydrazine of formula (VII) in the presence of a strong acid such as PPA.

Compounds of formula (II) wherein $R_3$ is chosen from either $-SO_2(C_{1-6}$ alkyl), $-SO_2$ phenyl, (C=O)($C_{1-6}$ alkyl),

and $R_5$-$R_8$ are selected from H, $C_{1-6}$ alkyl, $C_{1-6}$ alkoxy, halo, nitro, may be prepared according to the process outlined in Scheme 2.

Scheme 2

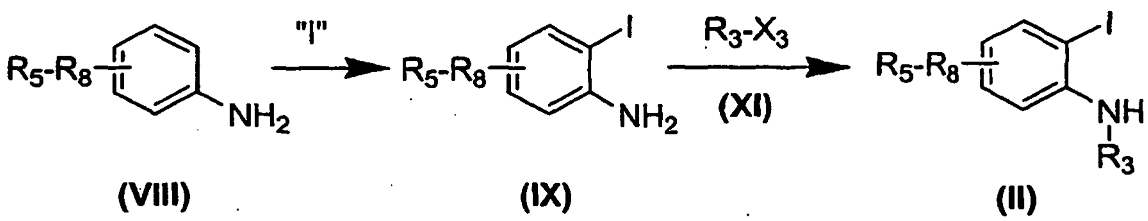


(VIII) (IX) (II)

A compound of formula (VIII), wherein $R_5$-$R_8$ are selected from H, $C_{1-6}$ alkyl, $C_{1-6}$ alkoxy, halo, nitro, a known compound or compound prepared by known methods, is treated with an iodinating agent such as N-iodo succinamide, Icl, or $I_2$ in a solvent such as acetic acid, acetonitrile, or the like, to yield the corresponding compound of formula (IX). The compound (IX), a known compound or compound prepared by known methods, is reacted with a compound of formula (XI), in which $R_3$ is chosen from either $-SO_2(C_{1-6}$ alkyl), - $SO_2$phenyl, (C=O)($C_{1-6}$ alkyl), (C=O)($C_{1-6}$ alkoxy), (C=O)phenyl, and $X_3$ is selected from Br, Cl, F, or a conventional activating anhydride, or ester, in the presence of a base such as pyridine, N,N-dimethyl aminopyridine, triethylamine, or sodium hydroxide in an organic solvent such as DCM, THF, or DMF to yield the corresponding compound of formula (II).

Compounds of formula (V) wherein W is OH, $NH_2$, $CO_2H$, and Ra-Re are selected from H, $C_{1-6}$ alkyl, $C_{1-6}$ alkoxy, halo, or nitro may be prepared according to the processes outlined in Scheme 3.

Scheme 3

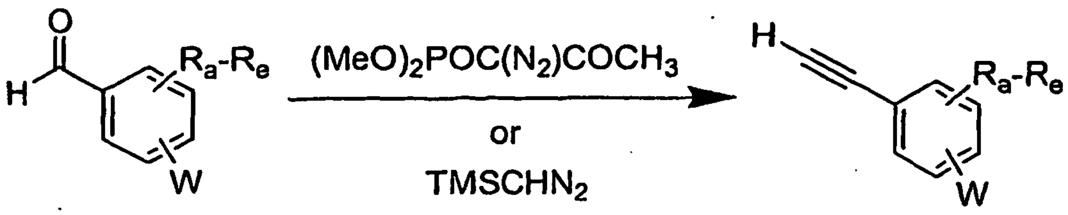


(X) (V)

A compound of formula (X) wherein W is OH, $NH_2$, $CO_2H$, and Ra-Re are selected from H, $C_{1-6}$ alkyl, $C_{1-6}$ alkoxy, halo, or nitro, is reacted with a diazophosphonate in the presence of a base such as, $K_2CO_3$, KOH, or DBU, in a solvent such as MeOH, EtOH, or DMF, to yield compounds of formula (V). Alternately compounds of formula (V) can also be prepared by treating a compound of formula (X) with trimethysilyldiazomethane, in the presence of a strong base such as LDA or LHMDS, in a solvent such as, THF, Ether, or MTBE, to yield compounds of formula (V). In addition compounds of formula (V) may also be obtained using methods known to one skilled in the art as outlined in R. C. Larock "Comprehensive Organic Transformations", VCH Publishers, 1989, p. 295-296.

Specifically compounds of formula (IV) wherein W is -OH, $-NH_2$, - C(O)OH may be prepared according to the processes outlined in Scheme 4.

Scheme 4

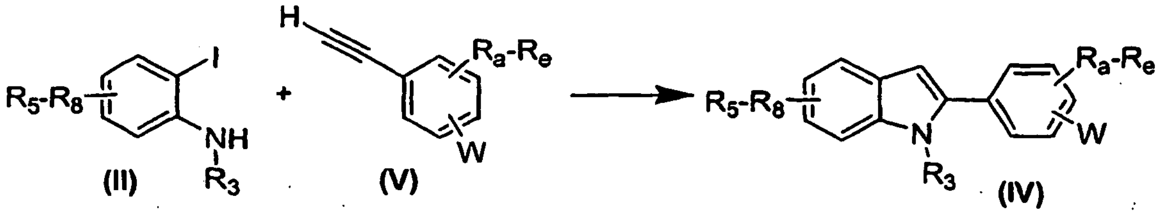

(II) (V) (IV)

Specifically, Compounds of formula (II), as defined in Scheme 2, are combined with compounds of formula (V), as defined in Scheme 3, in the presence of a palladium catalyst such as, $Pd(PPh_3)_2Cl_2$, or $Pd(OAc)_2$, and a copper source such as CuI, CuOAc, or CuBr, and a base such as triethylamine or pyridine, in a solvent such as THF or DMF, to provide the corresponding compounds of formula (IV).

Compounds of formula (I) in which n is a whole number between 0 and 4, and Z is as described in claim (1), and $R_5$-$R_8$ and Ra-Re are as described in Scheme 4, can be obtained by the procedures described in Scheme 5-7.

Scheme 5

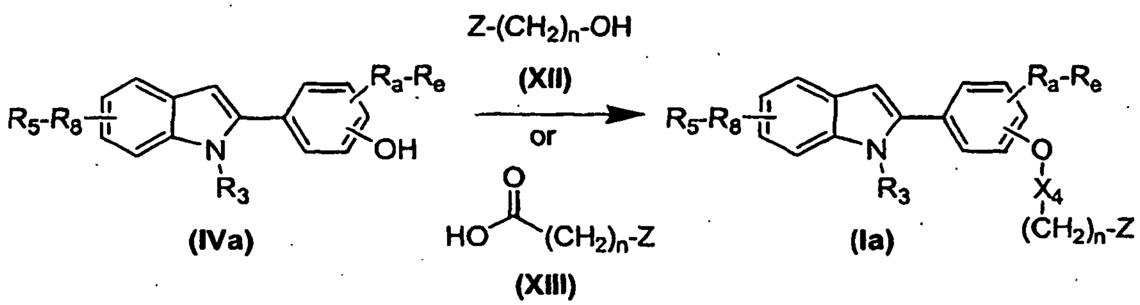


(IVa) (XIII) (Ia)

Specifically a compound of formula (IVa) wherein $R_3$, $R_a$-$R_e$ and $R_5$-$R_8$ are as described in Scheme 4, is reacted with an alcohol of formula (XII), wherein Z is as described in claim (1), and n is a whole number between 0 and 4, in the presence of a phosphine such as triphenyl phosphine, polymer supported triphenylphosphine, or tributylphosphine, and an azodicarboxylate such as diisopropylazodicarboxylate, 1,1'-(azodicarbonyl)dipiperidine, or other Mitsunobu conditions, in a solvent such as DCM or THF, to afford the corresponding compounds of formula (I) in which $X_4$ is a covalent bond, and n is a whole number between 0 and 4.

Alternatively compounds of formula (IV) as described above, can be reacted with carboxylic acids of formula (XIII), in which Z is defined as above, and n is a whole number between 0 and 3, in the presence of an activating agent such as carbonyldiimidazole or thionyl chloride, with a base such as N-methyl morpholine, triethylamine, or N,N-dimethyl-4-aminopyridine to yield the corresponding compound of formula (I), in which $X_4$ is defined as a carbonyl group.

Alternatively compounds of formula (Ib) can be obtained by the methods described in Scheme 6.

## Scheme 6

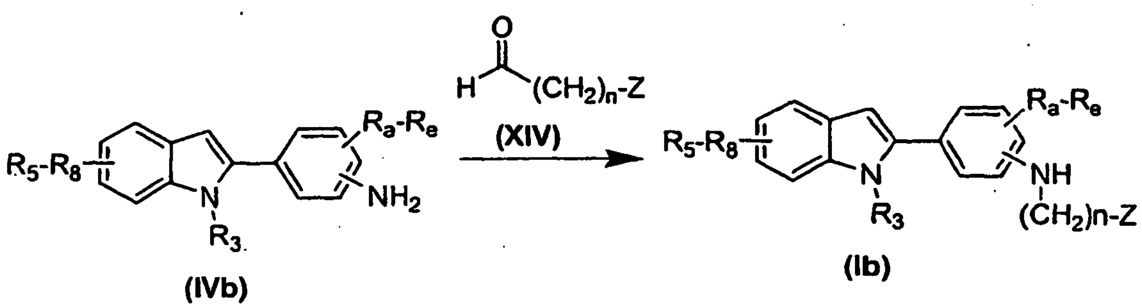


(IVb) (Ib)

Specifically, a compound of formula (IVb) in which $R_3$, $R_5$-$R_8$ and $R_a$-$R_e$ is as defined in scheme 4, is reacted with an aldehyde of formula (XIV) in which n is a whole number between 0 and 3, and Z is as described in claim (1), in the presence of a reducing agent such as $NaBH_3(CN)$ or $NaBH(OAc)_3$, in a solvent such as MeOH or THF, to afford the corresponding compound of formula (I).

Alternatively, compounds of formula (Ic) can be obtained using the methods outlined in Scheme 7.

## Scheme 7

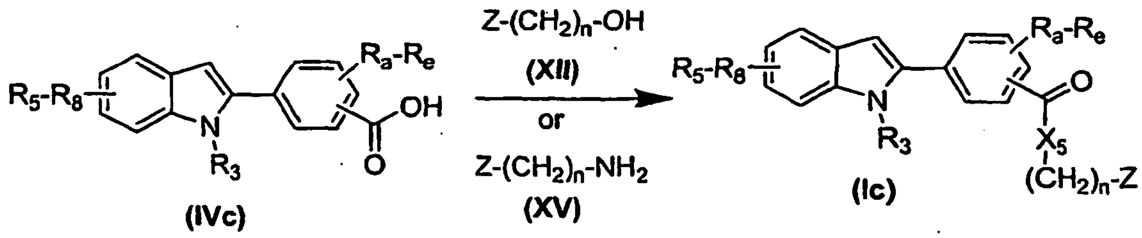


(IVc) (Ic)

Specifically, a compound of formula (IVc) in which $R_3$, $R_5$-$R_8$, and Ra-Re is defined as in Scheme 4, is reacted with an alcohol of formula (XII), or an amine of formula (XV), in which Z is as defined in Claim (1), and n is a whole number between 0 and 4, in the presence of an activating agent such as carbonyldiimidazole or thionyl chloride, with a base such as N-methyl morpholine, triethylamine, or N,N-dimethyl-4-aminopyridine to yield the corresponding compound of formula (I), in which $X_5$ is defined as O or NH.

In addition, compounds of formula (I)B can be converted to other compounds of formula (I)B as defined in Scheme 8 below.

Scheme 8

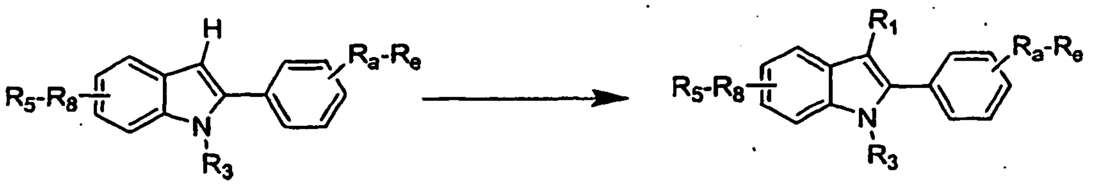

(Id) (Ie)

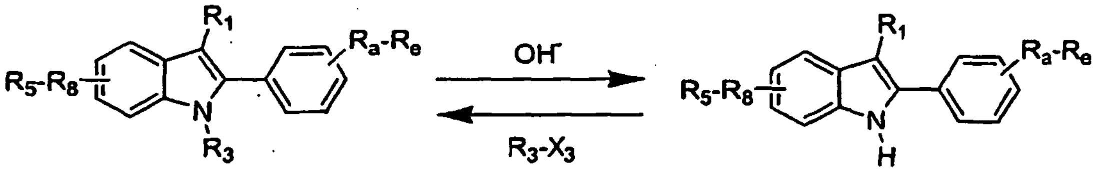


(Ie) (XI) (If)

Specifically, a compound of formula (Id) in which $R_3$, $R_5$-$R_8$, and $R_a$-$R_e$ are as described in Scheme 4, is treated with; a nitrating agent such as $HNO_3$ or an electrophilic halogenating agent such as $Br_2$ or NIS, using solvents and conditions known to one skilled in the art, to yield the corresponding compound of formula (If) in which $R_1$ is defined as $NO_2$, Br, Cl, or I. Additionally a compound of formula (If) in which $R_1$ is $NO_2$ can be further elaborated through reduction with an appropriate reducing agent such as $SnCl_2$ or iron metal, to yield the corresponding compound of formula (Ie), in which $R_1$ is $NH_2$.

Additionally, compounds of formula (Ie) in which $R_3$ is defined as in Scheme 2, and $R_5$-$R_8$, and $R_a$-$R_e$ are as described in Scheme 4, can be treated with a strong base such as KOH, $K_2CO_3$, or the like, in a solvent such as THF, MeOH, or the like, to yield the corresponding compounds of formula (If).

In addition, compounds of formula (If) can be converted to compounds of the corresponding formula (Ie) by treatment with a strong base such as n-BuLi, NaH, or the like, and an alkylating or acylating agent of formula (XI), wherein $R_3$ and $X_3$ are as defined in Scheme 2.

The synthesis of compounds of formula (III) in which Ra-Re are as defined in Scheme 3, $R_{11}$, and $R_{12}$ are as defined claim (1), and n is an integer from 2 to 5, and compounds of formula (IV) in which the above definitions apply, and $R_{13}$ is $C_{1-6}$ alkyl are described in Scheme 9.

Scheme 9

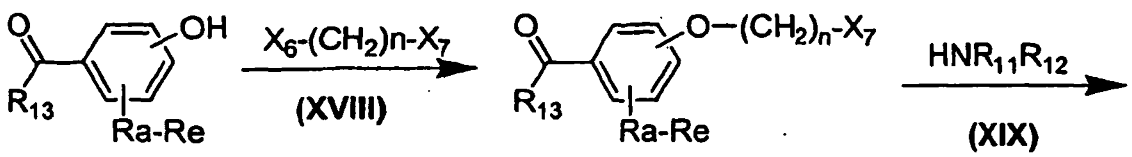


(XVI) (XVII)

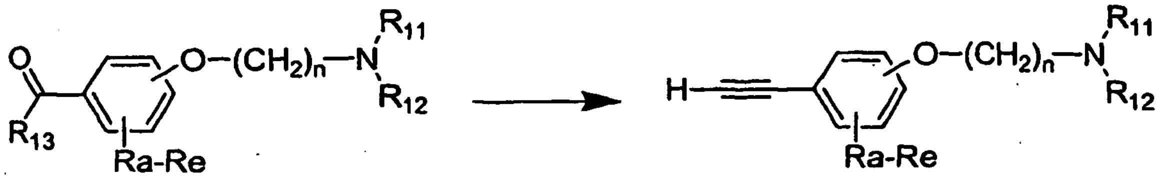

(VI) (III)

Specifically, a compound of formula (XVI) in which $R_{13}$ is H, or $C_{1-6}$ alkyl, and $R_a$-$R_e$ is as previously described, is treated with a base such as NaH or $K_2CO_3$, and a compound of formula (XVIII), in which $X_6$ is selected from Cl, Br, I, $-OSO_2CH_3$, -OTs, or OTf, and $X_7$ is selected from the same definition as $X_6$ but less reactive than the element chosen for $X_6$, and n is an integer from 2 to 5, in a solvent such as THF, DMF or DMSO, to yield the corresponding compound of formula (XVII). The compound of formula (XVII) is then treated with a compound of formula (XIX), wherein $R_{11}$ and $R_{12}$ are as defined in Claim (1), in a solvent such as DMF or DCM, to afford the corresponding compound of formula (VI).

Compounds of formula (III) are prepared by treatment of corresponding compounds of formula (IV), in which $R_{13}$ is defined as H, with a base such as LDA or LIHMDS, and $TMSCHN_2$, in a solvent such as THF, diethylether, or the like. Alternately, compounds of formula (III) can also be prepared by treating corresponding compounds of formula (IV), with a base such as $K_2CO_3$ or KOH, and a phosphonate such as $(CH_3O)_2P(O)C(N_2)C(O)CH_3$, in a solvent such as MeOH.

Compounds of formule (Ig) can be obtained using the methods described in Scheme 10.

Scheme 10

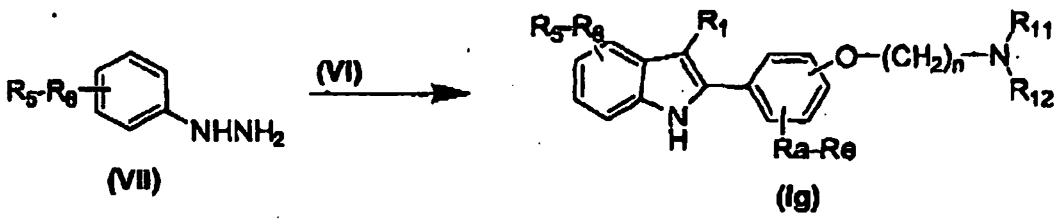


Specifically a compound of formula (VI) as defined in scheme 9 is treated with an aryl hydrazine of formula (VII), wherein $R_5$-$R_8$ is as defined as in claim (1), in polyphosphoric acid, to yield the corresponding compound of formula (Ig).

Additionally compounds of formula (I) can be formed using the procedures outlined In Scheme 11.

Scheme 11

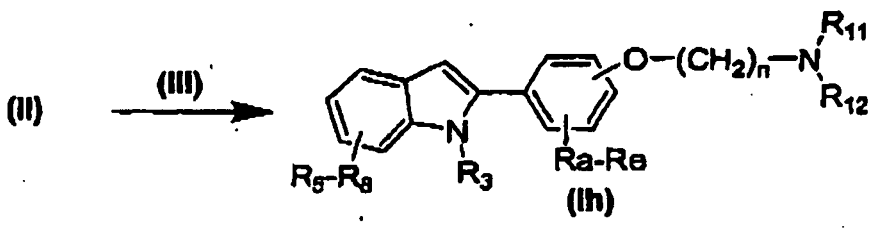


Specifically a compound of formula (II), as defined in Scheme 2 is combined with a compound of formula (III) as defined in Scheme 9, in the presence of a palladium catalyst such as $Pd(PPh_3)_2Cl_2$ or $Pd(OAc)_2$, and a copper source such as CuI, CuOAc or CuBr, and a base such as triethylamine or pyridine, in a solvent such as THF or DMF, to provide the corresponding compounds of formula (Ih).

D. Uses

According to the invention, the disclosed compounds and compositions are useful in the manufacture of medicaments for the amelioration of symptoms associated with, the treatment of, and/or the prevention of, the following conditions and diseases, or symptoms associated with them: dementia, Alzheimer's disease, narcolepsy, eating disorders, motion sickness, vertigo, attention deficit hyperactivity disorder, learning and memory disorders, schizophrenia, mild cognitive impairment upper airway allergic response (allergic rhinitis), insomnia, jet lag, obesity, asthma, neurogenic inflammation, substance abuse, bipolar disorders, manic disorders, and depression. The invention also features pharmaceutical compositions, which include, without limitation, one or more of the disclosed compound and a pharmaceutically acceptable carrier or excipient.

1. Dosages

Those skilled in the art will be able to determine, according to known methods, the appropriate dosage for a patient, taking into account factors such as age, weight, genenal health, the type of symptoms requiring treatment, and the use of other medications. An effective amount means that amount of pharmaceutical reagent (such as a prodrug, metabolic precursor, or active compound) that elicits the biological or medical response desired. In general, a therapeutically effective amount will be between 0.01 and 1000 mg/kg per day, preferably between 0.01 and 250 mg/kg body weight, and daily dosages will be between 0.50 and 5000 mg for an adult subject of normal weight. Capsules, tablets or other formulations (such as liquids and film-coated tablets) may be of between 0.20 and 100 mg, such as 0.20, 0.50, 1.0, 2.0, 3.0, and 10 mg and can be administered according to the disclosed uses.

2. Formulations

Dosage unit forms include tablets, capsules, pills, powders, granules, aqueous and nonaqueous oral solutions and suspensions, and parenteral solutions packaged In containers adapted for subdivision into individual doses. Dosage unit forms can also be adapted for various methods of administration. Including controlled release formulations, such as subcutaneous implants. Administration methods include oral, rectal, parenteral (intravenous, intramuscular, subcutaneous), intracisternal, intravaginal, intraperitoneal, intravesical, local (drops, powders, ointments, gels or cream), and by inhalation (a buccal or nasal spray) as appropriate depending on the overall health and condition of the patient as determined by a physician or veterinary doctor.

Parenteral formulations include pharmaceutically acceptable aqueous or nonaqueous solutions, dispersion, suspensions, emulsions, and sterile powders for the preparation thereof. Examples of carriers include water, ethanol, polyols (propylene glycol, polyethylene glycol), vegetable oils, and injectable organic esters such as ethyl oleate. Fluidity can be maintained by the use of a coating such as lecithin, a surfactant, or maintaining appropriate particle size. Carriers for solid dosage forme include (a) fillers or extenders, (b) binders, (c) humectants, (d) disintegrating agents, (e) solution retarders, (f) absorption accelerators, (g) adsorbants, (h) lubricants, (i) buffering agents, and (j) propellants.

Compositions may also contain adjuvants such as preserving, wetting, emulsifying, and dispensing agents; antimicrobial agents such as parabens, chlorobutanol, phenol, and sorbic acid; isotonic agents such as a sugar or sodium chloride; absorption-prolonging agents such as aluminum monostearate and gelatin; and absorption-enhancing agents.

3. Combination Therapy

The present invention also provides compositions useful in the manufacture of medicaments for the treatment of disorders or conditions modulated, preferably antagonized, by the histamine $H_2$ receptor in combination with compounds that modulate other receptors including, but not limited to, histamine $H_1$ and histamine $H_2$ receptors. The present invention includes compounds and compositions useful in the manufacture of medicaments for combination therapy for the treatment of diseases or conditions modulated by the histamine $H_3$ receptor in combination with compounds that are selective serotonin re-uptake inhibitors (SSRIs), such as PROZAC™, or are selective norepinephrine uptake inhibitors. Such combination therapy include (a) administering the two or more pharmaceutical agents separately formulated and at separate times, and (b) administering the two or more agents simultaneously in a single formulation or in separate formulations administered more or less at the same time. For example, one combination therapy comprising administering at least one histamine $H_5$ receptor modulating compound disclosed herein and administering at least one compound selected from a histamine H, receptor modulating compound, a histamine $H_2$ receptor modulating compound, a selective serotonin reuptake inhibitor (such as PROZAC™), or a selective norepinephrine uptake inhibiting compound.

4. Related Compounds

The invention provides the disclosed compounds and closely related, pharmaceutically acceptable forms of the disclosed compounds, i.e. salts, or hydrates thereof; and racemic mixtures, or enantiomerically or optically pure forms.

Pharmaceutically acceptable salts, include carboxylate salts (e.g., C $_{1-8}$alkyl, cycloalkyl, aryl, heteroaryl, or non-aromatic heterocyclic) amino acid addition salts, which are within a reasonable benefit/risk ratio, pharmacologically effective and suitable for contact with the tissues of patients without undue toxicity, Irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine, See example, S.M. Berge, et al., "Pharmaceutical Salts," J. Pharm. Sci., 1977, 66:1-19. Pharmaceutically acceptable amides of the

compounds of the invention may be derived from ammonia, primary C $_{1-6}$alkyl amines and secondary di (C $_{1-6}$alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C $_{1-3}$ alkyl primary amines, and di (C $_{1-2}$ alkyl)amines. Parmaceutically acceptable esters of the compounds of the invention may be derived from C $_{1-7}$ alkyl, C $_{5-7}$ cycloalkyl, phenyl, and phenyl($C_{1-6}$)alkyl esters. Preferred esters include methyl esters.

Also disclosed are compounds having one or more functional groups (e.g., hydroxyl, amino, or carboxyl) masked by a protecting group. See, e.g., Greene and Wuts, Protective Groups in Organic Synthesis, 3rd ed., (1999) John Wiley & Sons, NY. Some of these masked or protected compounds are pharmaceutically acceptable; others will be useful as intermediates.

HYDROXYL PROTECTING GROUPS

Protection for the hydroxyl group includes methyl ethers, substituted methyl ethers, substituted ethyl ethers, substitute benzyl ethers, and silyl ethers.

Substituted Methyl Ethers

Examples of substituted methyl ethers include methyoxymethyl, methylthiomethyl, *t*-butylthiomethyl, (phenyldimethylsilyl)methoxymethyl, benzyloxymethyl, p-methoxybenzyloxymethyl, (4-methoxyphenoxy)methyl, guaiacolmethyl, *t*-butoxymethyl, 4-pentenyloxymethyl, siloxymethyl, 2-methoxyethoxymethyl, 2,2,2-trichloroethoxymethyl, bis(2-chloroethoxy)methyl, 2-(trimethylsilyl)ethoxymethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S, S-dioxido, 1-[(2-chloro-4-methyl)phenyl]-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

Substituted Ethyl Ethers

Examples of substituted ethyl ethers include 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, and benzyl.

Substituted Benzyl Ethers

Examples of substituted benzyl ethers include *p*-methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, 2- and 4-picolyl, 3-methyl-2-picolyl N-oxido, diphenylmethyl, *p*, *p*'-dinitrobenzhydryl, 5-dibenzosuberyl, triphenylmethyl, $\alpha$-naphthyldiphenylmethyl, *p*-methoxyphenyldiphenylmethyl, di(p-methoxyphenyl)phenylmethyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris (benzoyloxyphenyt)methyl, 3-(*I*midazol-1-ylmethyl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, and benzisothiazolyl S,S-dioxido.

Silyl Ethers

Examples of silyl ethers include trimethylsilyl, triethylsilyl, triisopropylsilyl, dimethylisopropylsilyl, diethylisopropylsilyl, dimethylthexylsilyl, *t*-butyldimethylsilyl, *t*-butyldiphenylsilyl, tribenzylsilyl, tri-*p*-xylyisilyl, triphenylsilyl, diphenylmethylsilyl, and *t*-butylmethoxyphenylsilyl.

Esters

In addition to ethers, a hydroxyl group may be protected as an ester. Examples of esters include formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, *p*-P-phenylacetate, 3-phenylpropionate, 4-oxopentanoate (levulinate), 4,4-(ethylenedithio)pentanoate, pivaloate, adamantoate, crotonate, 4-methoxycrotonate, benzoate, *p*-phenylbenzoate, 2,4,6-trimethylbenzoate(mesitoate)

Carbonates

Examples of carbonate protecting groups include methyl, 9-fluorenylmethyl, ethyl, 2,2,2-trichloroethyl, 2-(trimethylsilyl)ethyl, 2-(phenylsulfonyl)ethyl, 2-(triphenylphosphonio)ethyl, isobutyl, vinyl, allyl, *p*-nitrophenyl, benzyl, *p*-methoxybenzyl, 3,4-dimethoxybenzyl, *o*-nitrobenzyl, *p*-nitrobenzyl, S-benzyl thiocarbonate, 4-ethoxy-1-naphthyl, and methyl dithiocarbonate.

Assisted Cleavage

Examples of assisted cleavage include 2-iodobenzoate, 4-azidobutyrate, 4-nitro-4-methylpentanoate, *o*-(dibromomethyl)benzoate, 2-formylbenzenesulfonate, 2-(methylthiomethoxy)ethyl carbonate, 4-(methylthiomethoxy)butyrate, and 2-(methylthiomethoxymethyl)benzoate.

Miscellaneous Esters

Examples of miscellaneous esters include 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate(tigloate), *o*-(methoxycarbonyl)benzoate, *p*-P-benzoate, α-naphthoate, nitrate, alkyl N,N,N',N'-tetramethylphosphorodiamidate, N-phenylcarbamate, borate, dimethylphosphinothioyl, and 2,4-dinitrophenylsulfenate

Sulfonates

Examples of sulfonates include sulfate, methanesulfonate(mesylate), benzylsulfonate, and tosylate.

AMINO PROTECTING GROUPS

Protection for the amino group includes carbamates, amides, and special -NH protective groups.

Examples of carbamates include methyl and ethyl carbamates, substituted ethyl carbamates, assisted cleavage carbamates, photolytic cleavage carbamates, urea-type derivatives, and miscellaneous carbamates.

Carbamates

Examples of methyl and ethyl carbamates include methyl and ethyl, 9-fluorenylmethyl, 9-(2-sulfo)fluorenylmethyl, 9-(2,7-dibromo)fluorenylmethyl, 2,7-di-*t*-butyl-[9-(10,10-dioxo-10,10,10,10-fetrahydrothioxanthyl)]methyl, and 4-methoxyphenacyl.

Substituted Ethyl

Examples of substituted ethyl carbamates include 2,2,2-trichloroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, 2-(N,N-dicyclohexylcarboxamido)ethyl, *t*-butyl, 1-adamantyl, vinyl, allyl, 1-isopropylallyl, cinnamyl, 4-nitrocinnamyl, 8-quinolyl, N-hydroxypiperidinyl, alkyldithio, benzyl, *p*-methoxybenzyl, *p*-nitrobenzyl, *p*-bromobenzyl, *p*-chlorobenzyl, 2,4-dichlorobenzyl, 4-methylsulfinylbenzyl, 9-anthrylmethyl and diphenylmethyl.

Assisted Cleavage

Examples of assisted cleavage include 2-methylthioethyl, 2-methylsulfonylethyl, 2-(*p*-toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, *m*-chloro-*p*-acyloxybenzyl, *p*-(dihydroxyboryl)benzyl, 5-benzisoxazolylmethyl, and 2-(trifluoromethyl)-6-chromonylmethyl.

Photolytic Cleavage

Examples of photolytic cleavage include *m*-nitrophenyl, 3,5-dimethoxybenzyl, *o*-nitrobenzyl, 3,4-dimethoxy-6-nitrobenzyl, and phenyl(*o*-nitrophenyl)methyl.

Urea-Type Derivatives

Examples of urea-type derivatives include phenothiazinyl-(10)-carbonyl derivative, N' -*p*-toluenesulfonylaminocarbonyl, and N'-phenylaminothiocarbonyl.

Miscellaneous Carbamates

Examples of miscellaneous carbamates include *t*-amyl, S-benzyl thiocarbamate, *p*-cyanobenzyl, cyclobutyl, cyclohexyl, cyclopentyl, cyclopropylmethyl, *p*-decyloxybenzyl, diisopropylmethyl, 2,2-dimethoxycarbonylvinyl, *o*-(N,N-dimethylcarboxamido)benzyl, 1,1-dimethyl-3-(N,N-dimethylcarboxamido)propyl, 1,1-dimethylpropynyl, di(2-pyridyl)methyl, 2-furanylmethyl, 2-iodoethyl, isobomyl, isobutyl, isonicotinyl, *p*-(*p*'-methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p*-phenylazophenyl)ethyl, 1-methyl-1-phenylethyl, 1-methyl-1-(4-pyridyl)ethyl, phenyl, *p*-(phenylazo)benzyl, 2,4,6-tri-*t*-butylphenyl, 4-(trimethylammonium)benzyl, and 2,4,6-trimethylbenzyl.

Examples of amides include:

Amides

N-formyl, N-acetyl, N-chloroacetyl, N-trichloroacetyl, N-trifluoroacetyl, N-phenylacetyl, N-3-phenylpropionyl, N-picolinoyl, N-3-pyridylcarboxamide, N-benzoylphenylalanyl derivative, N-benzoyl, N-p-phenylbenzoyl.

Assisted Cleavage

N-*o*-nitrophenylacetyl, N-*o*-nitrophenoxyacetyl, N-acetoacetyl, (N'-dithiobenzyloxycarbonylamino)acetyl, N-3-(*p*-hydroxyphenyl)propionyl, N-3-(*o*-nitrophenyl)propionyl, N-2-methyl-2-(*o*-nitrophenoxy)propionyl, N-2-methyl-2-(*o*-phenylazophenoxy)propionyl, N-4-chlorobutyryl, N-3-methyl-3-nitrobutyryl, N-*o*-nitrocinnamoyl, N-acetylmethionine derivative, N-*o*-nitrobenzoyl, N-*o*-(benzoyloxymethyl)benzoyl, and 4,5-diphenyl-3-oxazolin-2-one.

Cyclic Imide Derivatives

N-phthalimide, N-dithiasuccinoyl, N-2,3-diphenylmaleoyl, N-2,5-dimethylpyrrolyl, N-1,1,4,4-tetramethyldisilylazacyclopentane adduct, 5-substituted 1,3-dimethyl-1,3,5-triazacyclohexan-2-one, 5-substituted 1,3-dibenzyl-1,3,5-triazacyclohexan-2-one, and 1-substituted 3,5-dinitro-4-pyridonyl.

SPECIAL - NH PROTECTIVE GROUPS

Examples of special NH protective groups include

N-Alkyl and N-Aryl Amines

N-methyl, N-allyl, N-[2-(trimethylsilyl)ethoxy]methyl, N-3-acetoxypropyl, N-(1-isopropyl-4-nitro-2-oxo-3-pyrrolin-3-yl), quaternary ammonium salts, N-benzyl, N-di(4-methoxyphenyl)methyl, N-5-dibenzosuberyl, N-triphenylmethyl, N-(4-methoxyphenyl)diphenylmethyl, N-9-phenylfluorenyl, N-2,7-dichloro-9-fluorenylmethylene, N-ferrocenylmethyl, and N-2-picolylamine N'-oxide.

Imine Derivatives

N-1,1-dimethylthiomethylene, N-benzylidene, N-*p*-methoxybenzylidene, N-diphenylmethylene, N-[(2-pyridyl) mesityl]methylene, and N-(N' ,N'-dimethylaminomethylene).

PROTECTION FOR THE CARBOXYL GROUP

Substituted Methyl Esters

Examples of substituted methyl esters include 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, phenacyl, *p*-bromophenacyl, α-methylphenacyl, *p*-methoxyphenacyl, carboxamidomethyl, and N-phthalimidomethyl.

2-Substituted Ethyl Esters

Examples of 2-substituted ethyl esters include 2,2,2-trichloroethyl, 2-haloethyl, ω-chloroalkyl, 2-(trimethylsilyl) ethyl, 2-methylthioethyl, 1,3-dithianyl-2-methyl, 2-(p-nitrophenylsulfenyl)ethyl, 2-(p-toluenesulfonyl)ethyl, 2-(2'-pyridyl) ethyl, 2-(diphenylphosphino)ethyl, 1-methyl-1-phenylethyl, *t*-butyl, cyclopentyl, cyclohexyl, allyl, 3-buten-1-yl, 4-(trimethylsilyl)-2-buten-1-yl, cinnamyl, α-methylcinnamyl, phenyl, *p*-(methylmercapto)phenyl and benzyl.

Substituted Benzyl Esters

Examples of substituted benzyl esters include triphenylmethyl, diphenylmethyl, bis(*o*-nitrophenyl)methyl, 9-anthrylmethyl, 2-(9,10-dioxo)anthrylmethyl, 5-dibenzosuberyl, 1-pyrenylmethyl, 2-(trifluoromethyl)-6-chromylmethyl, 2,4,6-trimethylbenzyl, *p*-bromobenzyl, *o*-nitrobenzyl, *p*-nitrobenzyl, *p*-methoxybenzyl, 2,6-dimethoxybenzyl, 4-(methylsulfinyl)benzyl, 4-sulfobenzyl, piperonyl, 4-picolyl and *p*-P-benzyl.

Silyl Esters

Examples of silyl esters include trimethylsilyl, triethylsilyl, *t*-butyldimethylsilyl, *i*-propyldimethylsilyl, phenyldimethylsilyl and di-*t*-butylmethylsilyl.

Activated Esters

Examples of activated esters include thiols.

Miscellaneous Derivatives

Examples of miscellaneous derivatives include oxazoles, 2-alkyl-1,3-oxazolines, 4-alkyl-5-oxo-1,3-oxazolidines, 5-alkyl-4-oxo-1,3-dioxolanes, ortho esters, phenyl group and pentaaminocobalt(III) complex.

Stannyl Esters

Examples of stannyl esters include triethylstannyl and tri-*n*-butylstannyl.

AMIDES AND HYDRAZIDES

Amides

Examples of amides include N,N-dimethyl, pyrrolidinyl, piperidinyl, 5,6-dihydrophenanthridinyl, *o*-nitroanilides, N-7-nitroindolyl, N-8-Nitro-1,2,3,4-tetrahydroquinolyl, and *p*-P-benzenesulfonamides.

Hydrazides

Examples of hydrazides include N-phenyl and N,N'-diisopropyl hydrazides.

E. Chemical Examples

Example 1

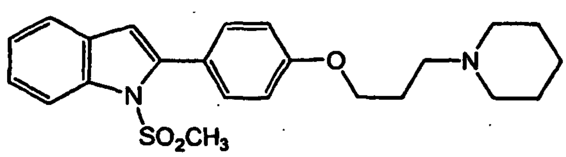

**2-[4-[3-Piperidinopropoxy]phenyl)-1-(methylsulfonyl)-1H-indole**
**$K_i$ = 7 nM**

Step A Preparation of 2-iodo-N-(methanesulfonyl)aniline

Methanesulfonyl chloride (4.4 mL, 57 mmol) was added to a 0°C dichloromethane (200 mL) solution containing 2-iodoaniline (5.0 g, 23 mmol) and triethylamine (9.6 mL, 69 mmol). The resulting mixture was stirred for 90 minutes, washed with HCl (0.5 M). The organics were separated then dried over sodium sulfate and concentrated in vacuo. The residue was then treated with potassium hydroxide (2.0 M in 1:1 methanol:water, 75 mL) for 30 min. This material was then diluted with dichloromethane and washed with HCl (1.0 N, 300 mL). The organics were separated then dried over sodium sulfate and concentrated to provide the title compound (5.15 g) as a tan solid.

Step B Preparation of 4-(methoxyethoxymethyl)benzaldehyde

Sodium hydride (2.4g (60%), 60 mmol) was added to 4-hydroxybenzaldehyde (6.0 g, 50 mmol) in N, N-dimethylformamide (100 mL). The suspension was stirred for 1 hour and then treated with 2-methoxyethoxymethyl chloride (6.8 mL, 60 mmol), and allowed to stir an additional 16 hours. The reaction was then partitioned between water and ether:ethyl acetate (1:1). The organics were then washed with water (4x), dried (potassium carbonate), and concentrated. The crude materials were then purified by silica gel chromatography (hexanes:ethyl acetate) to afford the title compound (9.0 g)

Step C Preparation of 1-ethynyl-4-(methoxyethoxymethyl)benzene

Dimethyl[(2-diazo-3-oxo)propyl] phosphonate was added in 4 portions to a suspension of potassium carbonate (4.96 g, 36 mmol), the product of Step B (3.78 g, 18 mmol), and methanol (50 mL). The reaction was stirred for 16 hours. and concentrated in vacuo. The residue was taken up in ether, washed with water (3x), dried (potassium carbonate), and concentrated. The crude product was purified by silica gel chromatography (hexane:ethyl acetate) to provide the title compound (2.3 g).

Step D Preparation of 2-(4-(methoxyethoxymethyl)phenyl)-1-(methanesulfonyl)indole

The products of Step A (3.0 g, 10 mmol) and step C (2.2 g, 10 mmol) were combined in N, N-dimethylformamide (20 mL) and triethylamine (5 mL). The solution was then treated with dichlorobis(triphenylphosphine)palladium(II) (0.7 g, 1.0 mmol), copper(I)iodide (380 mg, 2.0 mmol), and stirred at 80 °C for 17 hours. The reaction was then diluted with ether:ethyl acetate (1:1, 200 mL), washed with water (5x), dried (potassium carbonate), and concentrated in vacuo. The crude material was then purified by silica gel chromatography (hexane:ethyl acetate) to afford the title compound (3.36 g).

Step E Preparation of 2-(4-hydroxyphenyl)-1-(methanesulfonyl)-indole

A solution of the product of Step D (1.5 g, 4.0 mmol) in methanol (10 mL) was treated with HCl (10 mL, 4 N in dioxane). The reaction was allowed to stir for 2 hr, concentrated, and purified by silica gel chromatography (methanol: dichloromethane), to afford the title compound (0.93 g).

Step F Preparation of 2-[4-[3-piperidinopropoxy]phenyl]-1-(methanesulfonyl)indole

A mixture of immobilized triphenylphosphine resin (330 mg, 1.0 meq (Fluka)), and the product of Step E (140 mg, 0.50 mmol) in tetrahydrofuran (6.0 mL) was treated with 3-(piperdin-1-yl)propanol (143 mg, 1.0 mmol) followed by diethylazidodicarboxylate (0.16 mL, 1.1 mmol). The reaction was shaken for 20 hr. and filtered. The filtrate was concentrated in vacuo and the residue purified by silica gel chromatography (methanol/ethyl acetate) to afford pure title compound (97 mg). MS (ESI) m/z 413 ($MH^+$); $^1$H-NMR ($CDCl_3$) δ 8.13 (d, 1H), 7.59 (d, 1H), 7.50 (d, 2H), 7.30 (m, 2H), 6.97 (d, 2H), 6.68 (s, 1H), 4.08 (t, 2H), 2.72 (s, 3H), 2.4 (m, 6H), 1.63 (m, 6H), 1.45 (m, 2H).

Example 2 (for reference)

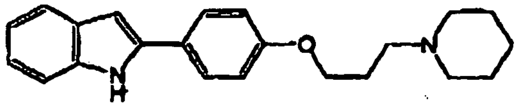

2-[4-[3-Piperidinopropoxy]phenyl)-1H-indole
$K_i$ = 48 nM

A solution of the product from Step F, Example 1 (41.4 mg, 0.10 mmol) in methanol (2.0 mL) was treated with potassium hydroxide (1.0 mL, 40% aq). The reaction was stirred at 40°C for 48 hours and concentrated In vacuo. The residue was purified by silica gel chromatography (methanol:dichloromethane) to provide pure title compound (3.7 mg). MS (ESI) m/z 335 ($MH^+$); $^1$H-NMR ($CDCl_3$) δ 8.19 (bs, 1H), 7.48 (d, 2H), 7.27 (m, 2H), 7.07 (t, 1H), 7.00 (t, 1H), 6.86 (d, 2H), 6.61 (s, 1H), 3.96 (t, 2H), 2.5 (m, 6H), 1.4 (m, 8H).

Example 3

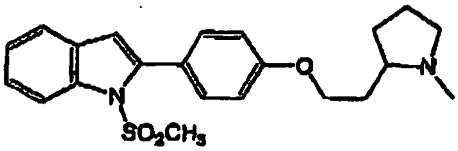

2-[4-[2-[1-(methyl)-2-pyrrolidinyl]ethoxy]phenyl)-1-(methylsulfonyl)-1H-indole
$K_i$ = 77 nM

The title compound was obtained (70 mg) by the same general method as Example 1 by substituting 2-ethoxy-1-methylpyrrolidine for 3-(piperdin-1-yl)propanol in step F. MS (ESI) m/z 399 ($MH^+$); $^1$H-NMR ($CDCl_3$) δ 8.13 (d, 1H), 7.60 (d, 1H), 7.50 (2, 2H), 7.37 (m, 2H), 6.97 (d, 2H), 6.68 (s, 1H), 4.08 (m, 2H), 3.12 (m, 1H), 2.72 (s, 3H), 2.39 (s, 3H), 2.1-1.7 (m, 8H).

Example 4 (for reference)

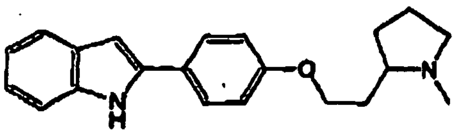

2-[4-[2-[1-(methyl)-2-pyrrolidinyl]ethoxy]phenyl)-1H-indole
$K_i$ = 100 nM

The title compound was obtained (14.3 mg) by the same general method as Example 2 by substituting the product of Example 3 for the product of example 1 as the starting material. MS (ESI) m/z 321 ($MH^+$); $^1$H-NMR ($CDCl_3$) δ 8.15 (bs, 1H), 7.49 (d, 1H), 7.47 (d, 2H), 7.26 (d, 1H), 7.04 (t, 1H), 6.98 (t, 1H), 6.85 (d, 2H), 6.59 (s, 1H), 3.96 (m, 2H), 2.96 (t, 1H), 2.23 (s, 3H), 2.1-1.7 (m, 8H).

Example 5

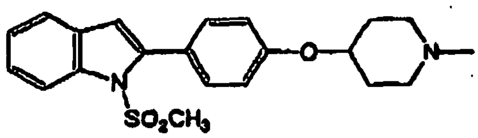

**2-[4-[1-(methyl)-4-piperidinyl]oxyphenyl]-1-(methylsulfonyl)-1H-indole**
**$K_i$ = 107 nM**

The title compound was obtained (54.8 mg) by the same general method as Example 1 by substituting 4-hydroxy-1-methylpiperidine for 3-(1-piperdinyl) propanol in Step F. MS (ESI) m/z 385 ($MH^+$); $^1$H-NMR ($CDCl_3$) δ 8.13 (d, 1H), 7.59 (d, 1H), 7.50 (d, 2H), 7.37 (m, 2H), 6.96 (d, 2H), 6.68 (s, 1H), 4.41 (m, 1H), 2.76 (m, 2H), 2.73 (s, 3H). 2.34 (s, 3H), 2.07 (m, 2H), 1.92 (m, 2H), 1.78 (m, 2H).

Example 6 (for reference)

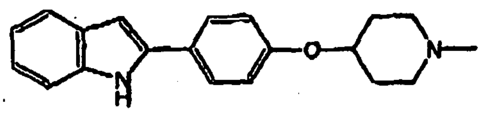

**2-[4-[1-(methyl)-4-piperidinyl]oxyphenyl] 1H-indole**
**$K_i$ = 390 nM**

The title compound was obtained (13.8 mg) by the same general method as Example 2 by substituting the product of Example 5 for the product of example 52 as the starting material. MS (ESI) m/z 307 ($MH^+$); $^1$H-NMR ($CDCl_3$) δ 8.36 (bs, 1H), 7.63 (d, 1H), 7.60 (d, 2H), 7.41 (d, 1H), 7.19 (t, 1H), 7.13 (t, 1H), 7.00 (d, 2H), 6.73 (s, 1H), 4.42 (m, 1H), 2.76 (m, 2H), 2.40 (m, 2H), 2.36 (s, 3H), 2.09 (m, 2H), 1.92 (m, 2H).

Example 7

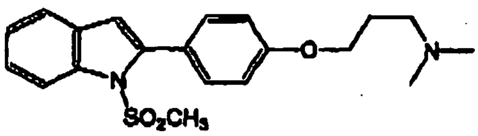

**2-[4-[3-Dimethylaminopropoxy]phenyl]-1-(methylsulfonyl)-1H-indole**
**$K_i$ = 120 nM**

The title compound was obtained (95 mg) by the same general method as Example 1 by substituting N, N-dimethyl-3-amino-1-propanol for 3-(piperdin-1-yl) propanol in Step F. MS (ESI) m/z 373 ($MH^+$); $^1$H-NMR ($CDCl_3$) δ 8.13 (d, 1H), 7.59 (d, 1H), 7.50 (d, 2H), 7.36 (m, 2H), 6.97 (d, 2H), 6.68 (s, 1H), 4.09 (t, 2H), 2.73 (s, 3H), 2.50 (t, 2H), 2.29 (s, 6H), 2.01 (m, 2H).

Example 8 (for reference)

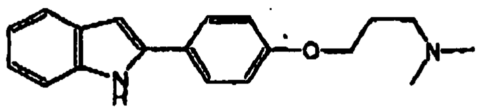

2-[4-(3-Dimethylaminopropoxy]phenyl] 1H-indole
$K_i$ = 500 nM

The title compound was obtained (13.8 mg) by the same general method as Example 2 by substituting the product of Example 7 for the product of Example 1 as the starting material. MS (ESI) m/z 295 ($MH^+$); $^1$H-NMR ($CDCl_3$) δ 8.10 (bs, 1H), 7.62 (d, 1H), 7.60 (d, 2H), 7.40 (d, 1H), 7.19 (t, 1H), 7.13 (t, 1H),7.00 (d, 2H), 6.72 (s, 1H), 4.09 (t, 2H), 2.50 (t, 2H), 2.29 (s, 6H), 2.01 (m, 2H).

Example 9

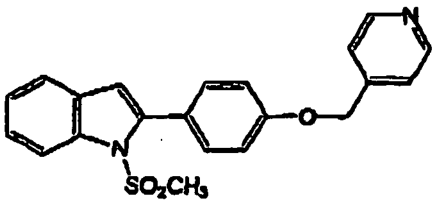

2-[4-[4-Pyridinyl]methoxyphenyl]-1-(methylsulfonyl)-1H-indole
$K_i$ = 6000 nM

The title compound was obtained (185 mg) by the same general method as Example 1 by substituting 4-hydroxymethylpyridine for 3-(piperdin-1-yl) propanol in Step F. MS (ESI) m/z 379 ($MH^+$); $^1$H-NMR ($CDCl_3$) δ 8.67 (d, 2H), 8.13 (d, 1H), 7.60 (d, 1H), 7.54 (d, 2H), 7.41 (d, 2H), 7.38 (m. 2H), 7.03 (d, 2H), 6.69 (s, 1H), 4.77 (s, 2H), 2.74 (s, 3H).

Example 10

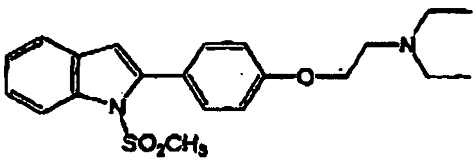

2-[4-[2-Diethylaminoethoxy]phenyl]-1-(methylsulfonyl)-1H-indole
$K_i$ = 369 nM

The title compound was obtained (140 mg) by the same general method as Example 1 by substituting 2-(N, N-diethylamino)ethanol for 3-(piperdin-1-yl) propanol in Step F. MS (ESI) m/z 387 ($MH^+$); $^1$H-NMR ($CDCl_3$) δ 8.14 (d, 1H), 7.59 (d, 1H), 7.50 (d, 2H), 7.18 (m, 2H), 6.97 (d, 2H), 6.87 (s, 1H), 4.12 (t, 2H), 2.91 (t, 2H), 2.73 (s, 3H), 2.87 (q, 4H), 1.10 (t, 6H).

Example 11 (for reference)

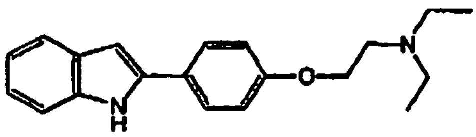

**2-{4-[2-Diethylaminoethoxy]phenyl]-1H-indole**
**$K_i$ = 523 nM**

The title compound from Example 10 (38.6 mg, 0.10 mmol) was treated with tetrabutyl ammonium fluoride (4.0 mL, of a 0.5 M in tetrahydrofuran) and stirred for 14 hours at 60°C. The resulting solution was concentrated in vacuo, dissolved in dichloromethane, and washed with water. The organics were then concentrated, and the crude product purified by silica gel chromatography (methanol/ dichloromethane) to afford pure title compound (5.9 mg). MS (ESI) m/z 309 ($MH^+$); $^1$H-NMR ($CDCl_3$) δ 8.21 (bs, 1H), 7.53 (d, 1H), 7.51 (d, 2H), 7.31 (d, 1H), 7.09 (t, 1H), 7.04 (t, 1H), 6.90 (d, 2H), 6.64 (s, 1H), 4.06 (t, 2H), 2.88 (t, 2H), 2.63 (q, 4H), 1.04 (t, 6H).

Example 12

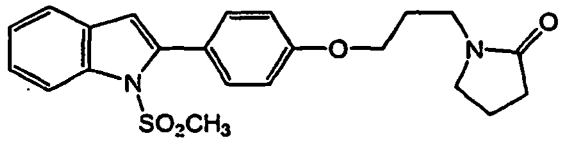

**2-[4-[3-(2-Oxo-pyrrolidino)propoxy]phenyl]-1-(methylsulfonyl)-1H-indole**

The title compound was obtained (180 mg) by the same general method as Example 1 by substituting 1-(3-hydroxypropyl)-2-pyrrolidineone for 3-(piperdin-1-yl) propanol in Step F. MS (ESI) m/z 435 (M+Na); $^1$H-NMR ($CDCl_3$) δ 8.12 (d, 1H), 7.59 (d, 1H), 7.49 (d, 2H), 7.36 (m, 2H), 6.95 (d, 2H), 6.67 (s, 1H), 4.03 (t, 2H), 3.55-3.35 (m, 6H), 2.72 (s, 3H), 2.40 (m, 2H), 2.06 (m, 2H).

Example 13

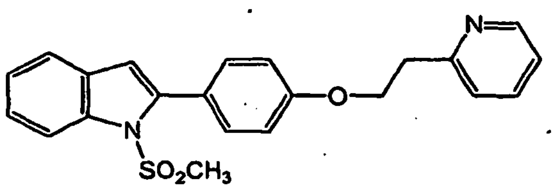

**2-[4-[2-(2-Pyridinyl)ethoxyphenyl]-1-(methylsulfonyl)-1H-indole**

The title compound was obtained (90 mg) by the same general method as Example 1 by substituting 2-(2-hydroxyethyl)pyridine for 3-(piperdin-1-yl)propanol in Step F. MS (ESI) m/z 393 ($MH^+$); $^1$H-NMR ($CDCl_3$) δ 8.59 (d, 1 H), 8.12 (d, 1H), 7.67 (d, 1H), 7.59 (d, 1H), 7.48 (d, 2H), 7.35 (m, 2H), 7.20 (m, 2H), 6.97 (d, 2H), 6.66 (s, 1H), 4.43 (t, 2H), 3.32 (t, 2H), 2.72 (s, 3H).

Example 14 (for reference)

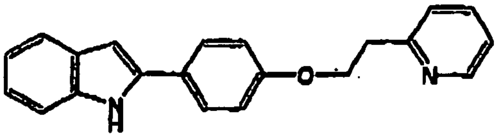

2-{4-[2-(2-Pyridinyl)ethoxyphenyl]-1H-indole

The title compound was obtained (14.5 mg) by the same general method as Example 11 by substituting the product of Example 13 for the product of Example 10 as the starting material. MS (ESI) m/z 315 ($MH^+$); $^1H$-NMR ($CDCl_3$) δ 8.52 (d, 1H), 8.45 (bs, 1H), 7.58 (t, 1H), 7.53 (d, 1H), 7.50 (d, 2H), 7.29 (d, 1H), 7.22 (d, 1H), 7.12 (t, 1H), 7.08 (t, 1H), 7.01 (t, 1H), 6.88 (d, 2H), 6.63 (s, 1H), 4.32 (t, 2H), 3.21 (t, 2H).

Example 15

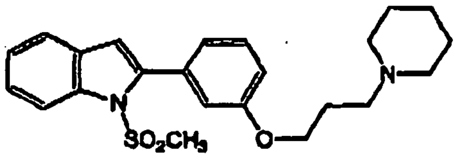

2-[3-[3-Piperidinopropoxy]phenyl)-1-(methylsulfonyl)-1H-indole
$K_i$ = 33 nM

The title compound was obtained (84 mg) by the same general method as Example 1 by substituting 3-hydroxybenzaldehyde for 4-hydroxybenzaldehyde in Step B. MS (ESI) m/z 413 ($MH^+$); $^1H$-NMR ($CDCl_3$) δ 8.14 (d, 1H), 7.62 (d, 1H), 7.37 (m, 3H), 7.16 (d, 1H), 7.12 (s, 1H), 6.98 (d, 1H), 6.74 (s, 1H), 4.08 (t, 2H), 2.77 (s, 3H), 2.62 (t, 2H), 2.66 (m, 2H), 2.10 (m, 2H), 1.69 (m, 4H), 1.50 (m, 1H).

Example 16 (for reference)

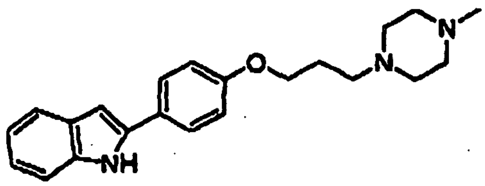

2-(4-(3-(4-Methylpiperazino)propoxy)phenyl)indole

$K_i$ = 2000 nM

Step A Preparation of 4'-(3-chloropropoxy)acetophenone

A solution of 4'-hydroxyacetophenone (20 mmol, 2.72 g), 3-bromopropionyl chloride (21 mmol, 2.07 mL) and potassium carbonate (4.14g, 30.0 mmol) in acetone (50 mL) was heated at reflux for overnight. The salt was filtered off. The solvent was evaporated. After drying in vacuo, the title compound (4.24 g) was collected.

Step B Preparation of 2-(4-(3-chloropropoxy)phenyl)indole

A mixture 4'-(3-chloropropoxy)acetophenone (10 mmol, 2.12 g) and phenylhydazine (10 mmol, 1.08 g) was heated at 100 °C for 40 min. Then PPA (~5 g) was added and temperature was raised to 130 °C and kept for 10 min.

The mixture was cooled down. Water (50 mL) was added. After 2 h, greenish solid was formed and collected via filtration. The solid then was washed by a small amount of methanol (5 mL). After drying in vacuo, the title compound (1.5 g) was obtained.

Step C 2-(4-(3-(4-Methylpiperazino)propoxy)phenyl)indole

The mixture of (2-(4-(3-chloropropoxy)phenyl)indole (1 mmol, 285 mg) and 4-methylpiperazine (2 mL) was heated at 80 °C for 5 h. After concentration, the residue was purified by column chromatography ($MeOH/CH_2Cl_2$) afforded the title compound (285 mg): $^1H$ NMR ($CDCl_3$, 400MHz) δ 8.26 (s, 1H), 7.53 (m, 3H), 7.32 (bd, 1H, *J* = 8.1 Hz), 7.1 (td, 1H, *J* = 1.1, 7.0 Hz), 7.05 (td, 1H, *J* = 1.1, 7.0 Hz), 6.90 (m, 2H), 6.64 (m, 1H), 3.99 (t, 2H, *J* = 6.3 Hz), 2.48 (m, 10H, *J* = 6.4 Hz), 2.25 (s, 3H), 1.93 (quintet, 2H, *J* = 6.3 Hz); EIMS *m*/*z* 350 ($M + H^+$).

Example 17

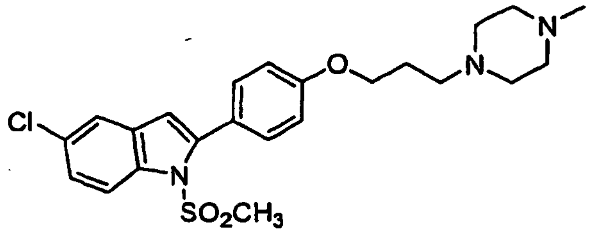

**1-(Methylsulfonyl)-2-(4-(3-(4-methylpiperazino)propoxy)phenyl)indole**

**$K_i$ =3000 nM**

Step A Preparation of 4-(3-chloropropoxy)benzaldehyde

A solution of 4-hydroxybenzaldehyde (100 mmol, 12.2 g), 3-bromopropionyl chloride (101 mmol, 20 mL) and potasium carbonate (20.7 g, 150 mmol) in acetone (250 mL) was heated at reflux for overnight. The salt was filtered off. The solvent was evaporated. Reduced pressure distillation provided the title compound (15 g).

Step B Preparation of 1-ethynyl-4-(3-chloropropoxy)benzene

To LDA (2M in THF, 15 mL) in THF (100 mL) at -78°C, $TMSCHN_2$ (2M in hexanes, 15 mL) was added dropwisely. Ten minutes later, 4-(3-chloropropoxy)benzaldehyde (0.025 mol, 4.97 g) in THF (60 mL) was added. After 1 h stirring at -78°C, the mixture was warmed up and refluxed for 3h. Water (250 mL) was added and extracted by EtOAc (2 x 200 mL). After being dried over $Na_2SO_4$ and concentration, the title compound (4.8 g) was obtained.

Step C Preparation of 1-ethylnyl-4-(3-(4-methylpiperazino)propoxy)benzene

The mixture of 1-ethynyl-4-(3-chloropropoxy)benzene (2 mmol, 388 mg) and 4-methylpiperazine (2 mL) was heated at 80 °C for 5h. After concentration, the residue was purified by column chromatography ($MeOH/CH_2Cl_2$) afforded the title compound (400 mg).

Step D Preparation of 2-iodo-N-(methanesulfonyl)aniline

To the mixture of the 2-iodo-4-chloroaniline (5.0 g, 20 mmol) and triethylamine (8.3 mL, 60 mmol) in methylene chloride (200 mL), the solution of methanesulfonyl chloride (3.4 mL, 44 mmol) was added slowly. The solution then was stirred at room temperature for 2 h. After being washed by HCl solution (0.5 N, 2 x100 mL), NaOH solution (0.5 N, 2 x 100 mL), brine (100 mL), the organic layer was dried over $Na_2SO_4$ and concentrated providing the title compound (6.6 g).

Step E Preparation of 1-(methy(sulfonyl)-2-(4-(3-(4-methylpiperazino)propoxy)phenyl)indole

The mixture of 1-ethylnyl-4-(3-(4-methylpiperazino)propoxy)benzene (230 mg, 0.89 mmol), 2-iodo-N-(methanesulfonyl)aniline (0.89 mmol, 296 mg), CuI (17 mg, 0.089 mmol), $Pd(PPh_3)_2Cl_2$ (32 mg, 0.045 mmol) and triethylamine (0.5 mL) in DMF (5 mL) was stirred at 80 °C for overnight. After concentration, water (20 mL) was added and extracted by methlene chloride (3 x 20 mL). The organics was concentrated and purified by column chromatography afforded the title compound (260 mg): $^1H$ NMR ($COCl_3$, 400MHz) δ 8.05 (d, 1H, *J* = 8.8 Hz), 7.55 (d, 1H, *J* = 2.1 Hz), 7.50 (td, 2H, *J* = 8.8, 2.1 Hz), 7.32 (d, 1H, *J* = 2.1 Hz), 7.30 (d, 1H, *J* = 2.1 Hz), 6.96 (td, 2H *J* = 2.1, 8.8 Hz), 6.60 (d, 1H, *J* = 0.5 Hz), 4.08 (t, 2H, *J* = 6.3 Hz), 2.74 (s, H), 2.55 (m, 10H), 2.33 (s, 3H), 2.02 (quintet, 2H, *J* = 6.3 Hz); EIMS *m*/*z* 462 ($M + H^+$).

F. Biological Examples

Biological Example 1

1(A) Transfection of cells with human histamine receptor

A 10 cm tissue culture dish with a confluent monolayer of SK-N-MC cells was split two days prior to transfection. Using sterile technique the media was removed and the cells were detached from the dish by the addition of trypsin. One fifth of the cells were then placed onto a new 10 cm dish. Cells were grown in a 37°C incubator with 5% $CO_2$ in Minimal Essential Media Eagle with 10% Fetal Bovine Serum. After two days cells were approximately 80% confluent. These were removed from the dish with trypsin and pelleted in a clinical centrifuge. The pellet was then re-suspended in 400 μL complete media and transferred to an electroporation cuvette with a 0.4cm gap between the electrodes (Bio-Rad #165-2088). One μg supercoiled $H_3$ receptor cDNA was added to the cells and mixed. The voltage for the electroporation was set at 0.25 kV, the capacitance is set at 960 μF.

After electroporation the cells were diluted into 10 mL complete media and plated onto four 10cm dishes. Due to the variability in the efficiency of electroporation, four different concentrations of cells were plated. The ratios used were: 1:20, 1:10, and 1:5, with the remainder of the cells being added to the fourth dish. The cells were allowed to recover for 24 hours before adding the selection media (complete media with 600 μg/ml G418). After 10 days dishes were analyzed for surviving colonies of cells. Dishes with well-isolated colonies were used. Cells from individual colonies were isolated and tested. SK-N-MC cells were used because they give efficient coupling for inhibition of adenylate cyclase. The clones that gave the most robust inhibition of adenylate cyclase in response to histamine were used for further study.

1(B) [3H]-N-methylhistamine binding

Cell pellets from histamine $H_3$ receptor-expressing SK-N-MC cells were homogenized in 20 mM TrisHCl/0.5mM EDTA. Supernatants from a 800 g spin were collected, reccentrifuged at 30,000 g for 30 minutes. Pellets were rehomogenized in 50 mM Tris/5mM EDTA (pH 7.4). Membranes were incubated with 0.8 nM [$^3H$]-N-methylhistamine plus/minus test compounds for 45 minutes at 25°C and harvested by rapid filtration over GF/C glass fiber filters (pretreated with 0.3% polyethylenimine) followed by four washes with ice cold buffer. Filters were dried, added to 4 mL scintillation cocktail and then counted on a liquid scintillation counter. Non-specific binding was defined with 10 μM histamine. $PK_I$ values were calculated based on a $K_D$ of 800 pM and a ligand concentration ([L]) of 800 pM according to the formula:

$$K_I=(IC_{50})/(1 + ([L]/(K_D))$$

**Claims**

**1.** A compound of formula (I)(B):

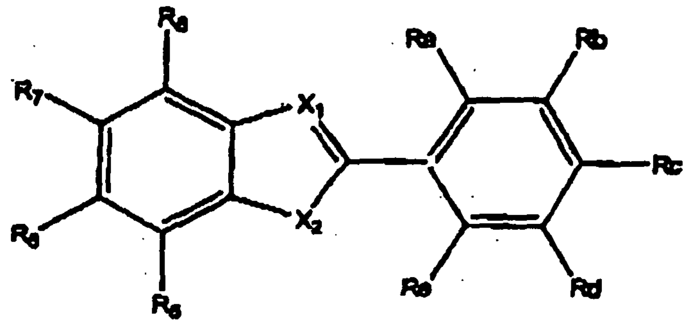

wherein

$X_1$ is $CR_1$, wherein $R_1$ is H, halo, cyano, amino, or nitro: and $X_2$ is $NR_3$;
$R_3$ is W'Z';
W' is (C=O) or $SO_2$;
Z' is C $_{1-6}$ alkyl, C $_{1-8}$ alkoxy, C $_{3-8}$ cycloalkyl, phenyl, or C $_{2-6}$ heterocyclic radical, optionally including in the ring up to 3 additional heteroatoms or moieties independently selected from O, N, NH, S, SO, and $SO_2$; or Z' is $NR_{13}R_{14}$ where each of $R_{13}$ and $R_{14}$ is independently selected from C $_{1-6}$ alkyl, C $_{2-6}$ alkenyl, phenyl, benzyl, C $_{3-6}$ cycloalkyl, and C $_{2-6}$ heterocyclic radical;
each of $R_5$, $R_6$, $R_7$ and $R_8$ is independently H, C $_{1-6}$ alkyl, C $_{1-8}$ alkoxy, halo, nitro, or amino;
one of $R_a$, $R_b$, $R_c$, $R_d$, and $R_e$ is WZ and the others are independently selected from H, C $_{1-6}$ alkyl, C $_{1-6}$ alkoxy, halo, nitro, and amino;
W is -O-, or O-$R_9$,
wherein $R_9$ is C $_{1-6}$ alkylene, C $_{2-6}$ alkynylene, C $_{2-6}$ alkenylene, phenylene, or C $_{2-5}$ heterocyclic bivalent radical;
Z is C $_{2-5}$ heterocyclic radical with at least one basic nitrogen atom in the ring, optionally including In the ring up to 3 additional hetaroatoms or moietiés independently selected from O. C=O, N, NH, NO, S, SO, and $SO_2$, wherein G is $R_{15}$, $COR_{15}$, $COOR_{15}$, $SO_2R_{15}$, $SO_2N$, $CSR_{15}$; or Z is $NR_{11}R_{12}$ where each of $R_{11}$ and $R_{12}$ is independently selected from H, $C_{1-4}$ alkyl, phenyl, benzyl, C $_{3-8}$ cycloalkyl, and $C_{2-6}$ heterocyclic radical; or $NR_{11}R_{12}$ taken together is a C $_{3-8}$ cyclocalkylimino radical; and $R_{15}$ is $C_{1-4}$ alkyl, alkyl, $C_{2-5}$ alkynyl, $C_{2-5}$ akenyl, $C_{3-7}$ cycloalkyl, and $C_{4-7}$ cycloalkenyl;
each of the above hydrocarbyl or heterocylic groups being optionally substituted with between 1 and 3 substituents selected from $C_{1-3}$ alkyl, $C_{1-3}$ alkoxy, halo, hydroxy, phenyl, and phenyl(C $_{1-3}$ alkyl); and wherein each of the above heterocyclic groups may be attached to the rest of the molecule by a carbon atom or a heteroatom;

or a pharmaceutically acceptable salt, hydrate thereof.

**2.** A compound of claim 1, wherein $R_3$ is -(C=O)$C_{1-4}$ alkyl.

**3.** A compound of claim 1, wherein $R_3$ is -$SO_2$($C_{1-3}$ alkyl).

**4.** A compound of claim 3 wherein $R_3$ is methylsulfonyl.

**5.** A compound of claim 1. wherein $R_a$ is WZ.

**6.** A compound of claim 1, wherein $R_b$, or $R_d$ is WZ.

**7.** A compound of claim 1, wherein W Is ethoxy, propoxy, or butoxy:

**8.** A compound of claim 1, wherein W is -O-.

**9.** A compound of claim 1, wherein one of $R_b$, $R_c$, and $R_e$, is WZ and the others are independently selected from H, methyl, ethyl, methoxy, ethoxy, amino, nitro, and halo; and $R_a$ and $R_d$ are each independently H or methyl.

**10.** A compound of claim 1, wherein at least two of the following apply: $R_c$ is WZ; W is propoxy or ethoxy; and Z is N-piperidino, 2-(N-methyl)pyrrolidino, or dimethylamino.

**11.** A compound of claim 1, wherein Z is pyrrolidiono, N-methyl-pyrrolidino, pyridyl, thiazoyl, piperidino, or $NR_{11}R_{12}$ where each of $R_{11}$ and $R_{12}$ is independently selected from H, $C_{1-6}$ alkyl, phenyl, benzyl, C $_{3-6}$ cycloalkyl, and C $_{2-5}$ heterocyclic radical or taken together with the N form a C $_{6-8}$ cycloalkylamino radical.

**12.** A compound of claim 1, wherein one of $R_b$, $R_c$, and $R_e$ is WZ and the others are independently selected from H, methyl, ethyl, methoxy, ethoxy, amino, and halo; and $R_c$ and $R_d$ are each independently H or methyl;
W is -O- or C $_{1-3}$ alkoxy;
Z is pyrrolidino, N-methylpyrrolidino, pyridyl, thiazoyl, piperidino, piperazino, N-methylpiperazino, or $NR_{11}R_{12}$, where each of $R_{11}$, and $R_{12}$ is independently selected from H, C $_{1-2}$ alkyl, phenyl, benzyl, C $_{3-8}$ cycloalkyl, and C $_{2-5}$ heterocyclic radical; each of $R_6$ and $R_7$ are each independently H, methyl, methoxy, or ethoxy: each of $R_5$ and $R_6$ is H.

**13.** A compound of claim 12, wherein $R_3$ is -$SO_2$ (C $_{1-6}$ alkyl).

**14.** A compound of claim 12, wherein $R_3$ is $SO_2$(phenyl)) and (C=O)(C $_{1-6}$ alkyl).

**15.** A compound of claim 12, selected from [2-[4-[2-[1-(methyl)-2-pyrrolldinyl]ethoxyl]phenyl]-1-(methylsulfonyl)-1H-indole, and 1-(methylsulfonyl)-2-(4-(3-(4-methylpiperazino)-propoxy)phenyl)indole; or a pharmaceutically acceptable salt, or hydrate thereof.

**16.** A compound of claim 12, selected from 2-[4-[3 Piperidinopropoxyl]phenyl)-1-(methytsulfonyl)-1H-indole, and and 2-[3-[3-Piperidinopropoxy]phenyl)-1-(methylsulfonyl)-1H-indole or a pharmaceutically acceptable salt, or hydrate thereof.

**17.** A pharmaceutical composition comprising a compound of formula (I)B as defined in claim 1, and a pharmaceutically acceptable carrier.

**18.** A pharmaceutical composition of claim 17, wherein said compound has a formula wherein: one of $R_b$, $R_c$, and $R_e$ is WZ and the others are independently selected from H, methyl, ethyl, methoxy, ethoxy, amino, and halo:

$R_a$ and $R_d$ are each independently H or methyl;
W is -O- or C $_{1-3}$ alkoxy;
Z is pyrrolidino, N-methylpyrrolidino, pyridyl, thiazoyl, piperidino, or $NR_{11}R_{12}$, where each of $R_{11}$ and $R_{12}$ is independently selected from H, C $_{1-2}$ alkyl, phenyl, benzyl, C $_{3-6}$ cycloalkyl, and C $_{2-5}$ heterocyclic radical; and $R_6$ and $R_7$ are each independently H, methyl, methoxy, or ethoxy.

**19.** A pharmaceutical composition of claim 21, wherein said compound has a formula selected from 2-[4-12-[1-(methyl)-2-pyrrolidinyl]ethoxy]phenyl)-1-(methylsulfonyl)-1H-indole; 2-[4-[3-Piperidinopropoxy]phenyl)-1 (methylsulfonyl)-1H-indole; 2-[3-[3-Pipperidinopropoxy]phenyl)-1-(methylsulfonyl)-1H-indole; and 1-(methylsulfonyl)-2-(4-(3-(4-methylpiperazino)-propoxy)phenyl)indople; or a pharmaceutically acceptable salt, or hydrate thereof.

**20.** The use of a compound according to claim 1 in the manufacture of a medicament for treating a patient with a central nervous system disorder.

**21.** The use according to claim 20, wherein said central nervous system disorder is selected from sleep/wake disorders, arousal/vigilance disorders, dementia, Alzheimer's disease, epilepsy, narcolepsy, eating disorders, motion sickness, vertigo, attention deficit hyperactivity disorder, learning and memory disorders, mild cognitive impairment, and schizophrenia.

**22.** The use according to claim 20, wherein said disorder is selected from sleep/wake disorders, arousal/vigilance disorders, attention deficit hyperactivity disorder, and learning and memory disorders.

**23.** The use of a compound according to claim 1 in the manufacture of a medicament for treating a patient with an upper airway allergic response.

**24.** The use according to any of claims 20 to 23, wherein said compound has a formula wherein: one of $R_b$. $R_c$, and $R_e$ is WZ and the others are independently selected from H, methyl, ethyl, methoxy, ethoxy, amino, and halo;
$R_a$ and $R_d$ are each independently H or methyl;
W is -O- or $C_{1-3}$ alkoxy;

Z is pyrrolidino, N-methylpyrrolldino, pyridyl, thiazoyl, piperidino, N-methylpiperazino or $NR_{11}R_{12}$ where each of $R_{11}$ and $R_{12}$ is independently selected from H, $C_{1-2}$ alkyl, phenyl, benzyl. $C_{3-8}$ cycloalkyl, and $C_{2-5}$ heterocyclic radical; and $R_6$ and $R_7$ are each independently H, methyl, methoxy, or ethoxy.

**25.** The use according to any of claims 20 to 23, wherein said compound has a formula selected from 2-[4-[2-[1-(methyl)-2-pyrrolidinyl]ethoxy]phenyl)-1-(methylsulfonyl)-1H-indole; 2-[4-[3-Piperidinopropoxy]phenyl)-1-(methylsulfonyl)-1H-indole; 2-[3-[3-Piperidinopropoxy]phenyl)-1-(methylsulfonyl)-1H-indole; and 1-(methylsulfonyl)-2-(4-(3-(4-methylpiperazino)propoxy)phenyl)indole; or a pharmaceutically acceptable salt, or hydrate thereof.

## Patentansprüche

**1.** Verbindung von Formel (I)(B):

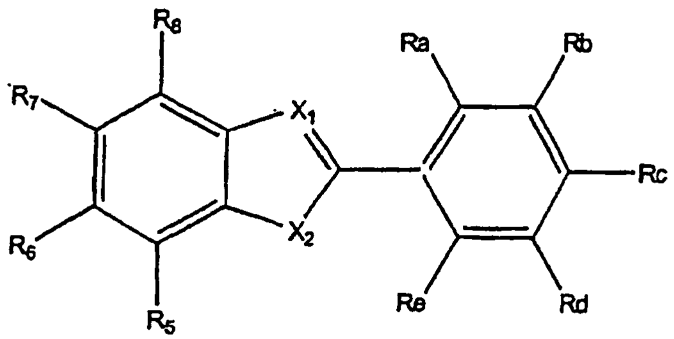

worin

$X_1$ $CR_1$ ist, worin $R_1$ H, Halo, Cyano, Amino oder Nitro ist; und $X_2$ $NR_3$ ist;
$R_3$ -W'Z' ist;
W' (C=O) oder $SO_2$ ist;
Z' $C_{1-6}$-Alkyl, $C_{1-6}$-Alkoxy, $C_{3-8}$-Cycloalkyl, Phenyl oder ein heterocyclischer $C_{2-6}$-Rest ist, gegebenenfalls einschließlich bis zu 3 zusätzlichen Heteroatomen oder -einheiten im Ring, die unabhängig ausgewählt sind aus O, N, NH, S, SO und $SO_2$;
oder Z' $NR_{13}R_{14}$ ist, worin $R_{13}$ und $R_{14}$ jeweils unabhängig ausgewählt ist aus $C_{1-6}$-Alkyl, $C_{2-6}$-Alkenyl, Phenyl, Benzyl, $C_{3-8}$-Cycloalkyl und einem heterocyclischen $C_{2-6}$-Rest;
$R_5$, $R_6$, $R_7$ und $R_8$ jeweils unabhängig H, $C_{1-6}$-Alkyl, $C_{1-6}$-Alkoxy, Halo, Nitro oder Amino ist;
eines von $R_a$, $R_b$, $R_c$, $R_d$ und $R_e$ WZ ist und die anderen unabhängig ausgewählt sind aus H, $C_{1-6}$-Alkyl, $C_{1-6}$-Alkoxy, Halo, Nitro und Amino;
W -O- oder O-$R_9$ ist, worin $R_9$ $C_{1-6}$-Alkylen, $C_{2-6}$-Alkinylen, $C_{2-6}$-Alkenylen, Phenylen oder ein heterocyclischer zweiwertiger $C_{2-5}$-Rest ist;
Z ein heterocyclischer $C_{2-8}$-Rest mit wenigstens einem basischen Stickstoffatom im Ring ist, gegebenenfalls einschließlich bis zu 3 zusätzlichen Heteroatomen oder -einheiten im Ring, die unabhängig ausgewählt sind aus O, C=O, N, NH, NG, S, SO und $SO_2$ wobei G $R_{15}$, $COR_{15}$, $COOR_{15}$, $SO_2R_{15}$, $SO_2N$, $CSR_{15}$ ist; oder Z $NR_{11}R_{12}$ ist, wobei $R_{11}$, und $R_{12}$ jeweils unabhängig ausgewählt ist aus H, $C_{1-6}$-Alkyl, Phenyl, Benzyl, $C_{3-8}$-Cycloalkyl und einem heterocyclischen $C_{2-5}$-Rest; oder $NR_{11}R_{12}$ zusammengenommen ein $C_{6-8}$-Cycloalkyliminorest ist; und $R_{15}$ $C_{1-6}$-Alkyl, $C_{2-6}$-Alkinyl, $C_{2-6}$-Alkenyl, $C_{3-7}$-Cycloalkyl und $C_{4-7}$-Cycloalkenyl ist; wobei jede der obigen Kohlenwasserstoff- oder heterocyclischen Gruppen gegebenenfalls mit zwischen 1 und 3 Substituenten substituiert sind, die ausgewählt sind aus $C_{1-3}$-Alkyl, $C_{1-3}$-Alkoxy, Halo, Hydroxy, Phenyl und Phenyl-($C_{1-3}$-alkyl); und wobei jede der obigen heterocyclischen Gruppen an den Rest des Moleküls durch ein Kohlenstoffatom oder ein Heteroatom gebunden sein kann;

oder ein pharmazeutisch verträgliches Salz oder Hydrat davon.

**2.** Verbindung nach Anspruch 1, wobei $R_3$ -(C=O)$C_{1-6}$-Alkyl ist.

**3.** Verbindung nach Anspruch 1, wobei $R_3$ -$SO_2$($C_{1-3}$-Alkyl) ist.

**4.** Verbindung nach Anspruch 3, wobei $R_3$ Methylsulfonyl ist.

**5.** Verbindung nach Anspruch 1, wobei $R_c$ WZ ist.

**6.** Verbindung nach Anspruch 1, wobei $R_b$ oder $R_d$ WZ ist.

**7.** Verbindung nach Anspruch 1, wobei W Ethoxy, Propoxy oder Butoxy ist.

**8.** Verbindung nach Anspruch 1, wobei W -O- ist.

**9.** Verbindung nach Anspruch 1, wobei eines von $R_b$, $R_c$ und $R_e$ WZ ist und die anderen unabhängig ausgewählt sind aus H, Methyl, Ethyl, Methoxy, Ethoxy, Amino, Nitro und Halo; und $R_a$ und $R_d$ jeweils unabhängig H oder Methyl sind.

**10.** Verbindung nach Anspruch 1, wobei wenigstens zwei von den folgenden zutreffen: $R_c$ ist WZ; W ist Propoxy oder Ethoxy; und Z ist N-Piperidino, 2-(N-Methyl)pyrrolidino oder Dimethylamino.

**11.** Verbindung nach Anspruch 1, wobei Z Pyrrolidino, N-Methylpyrrolidino, Pyridyl, Thiazolyl, Piperidino oder $NR_{11}R_{12}$ ist, wobei $R_{11}$ und $R_{12}$ jeweils unabhängig ausgewählt ist aus H, $C_{1-6}$-Alkyl, Phenyl, Benzyl, $C_{3-8}$-Cycloalkyl und einem heterocyclischen $C_{2-5}$-Rest oder zusammengenommen mit dem N einen $C_{6-8}$-Cycloalkylaminorest bilden.

**12.** Verbindung nach Anspruch 1, wobei eines von $R_b$, $R_c$ und $R_e$ WZ ist und die anderen unabhängig ausgewählt sind aus H, Methyl, Ethyl, Methoxy, Amino und Halo; und $R_a$ und $R_d$ jeweils unabhängig H oder Methyl sind;
W -O- oder $C_{1-3}$-Alkoxy ist;
Z Pyrrolidino, N-Methylpyrrolidino, Pyridyl, Thiazolyl, Piperidino, Piperazino, N-Methylpiperazino oder $NR_{11}R_{12}$ ist, wobei $R_1$ und $R_{12}$ jeweils unabhängig ausgewählt ist aus H, $C_{1-2}$-Alkyl, Phenyl, Benzyl, $C_{3-8}$-Cycloalkyl und einem heterocyclischen $C_{2-5}$-Rest; $R_6$ und $R_7$ jeweils unabhängig H, Methyl, Methoxy oder Ethoxy sind; $R_5$ und $R_8$ jeweils H ist.

**13.** Verbindung nach Anspruch 12, wobei $R_3$ -$SO_2$($C_{1-6}$-Alkyl) ist.

**14.** Verbindung nach Anspruch 12, wobei $R_3$ $SO_2$(Phenyl) und (C=O)($C_{1-6}$-Alkyl) ist.

**15.** Verbindung nach Anspruch 12, ausgewählt aus 2-[4-[2-[1-(Methyl)-2-pyrrolidinyl]ethoxy]phenyl)-1-(methylsulfonyl)-1H-indol und 1-(Methylsulfonyl)-2-(4-(3-(4-methylpiperazino)propoxy)phenyl)indol; oder einem pharmazeutisch verträglichen Salz oder Hydrat davon.

**16.** Verbindung nach Anspruch 12, ausgewählt aus 2-[4-[3-Piperidinopropoxy]phenyl)-1-(methylsulfonyl)-1H-indol und 2-[3-[3-Piperidinopropoxy]phenyl)-1-(methylsulfonyl)-1H-indol oder einem pharmazeutisch verträglichen Salz oder Hydrat davon.

**17.** Pharmazeutische Zusammensetzung, die eine Verbindung von Formel (I)B, wie definiert in Anspruch 1, und einen pharmazeutisch verträglichen Trägerstoff umfasst.

**18.** Pharmazeutische Zusammensetzung nach Anspruch 17, wobei die Verbindung eine Formel hat, in der: eines von $R_b$, $R_c$ und $R_e$ WZ ist und die anderen unabhängig ausgewählt sind aus H, Methyl, Ethyl, Methoxy, Ethoxy, Amino und Halo;
$R_a$ und $R_d$ jeweils unabhängig H oder Methyl sind;
W -O- oder $C_{1-3}$-Alkoxy ist;
Z Pyrrolidino, N-Methylpyrrolidino, Pyridyl, Thiazoyl, Piperidino oder $NR_{11}R_{12}$ ist, worin $R_{11}$und $R_{12}$ jeweils unabhängig ausgewählt ist aus H, $C_{1-2}$-Alkyl, Phenyl, Benzyl, $C_{3-8}$-Cycloalkyl und einem heterocyclischen $C_{2-5}$-Rest; und $R_6$ und $R_7$ jeweils unabhängig H, Methyl, Methoxy oder Ethoxy sind.

**19.** Pharmazeutische Zusammensetzung nach Anspruch 18, wobei die Verbindung eine Formel hat, die ausgewählt ist aus 2-[4-[2-[1-(Methyl)-2-pyrrolidinyl]ethoxy]phenyl)-1-(methylsulfonyl)-1H-indol; 2-[4-[3-Piperidinopropoxy]phenyl)-1-(methylsulfonyl)-1H-indol; 2-[3-[3-Piperidinopropoxy]phenyl)-1-(methylsulfonyl)-1H-indol; und 1-(Methylsulfonyl)-2-(4-(3-(4-methylpiperazino)propoxy)phenyl)indol; oder einem pharmazeutisch verträglichen Salz oder Hydrat davon.

**20.** Verwendung einer Verbindung nach Anspruch 1 zur Herstellung eines Arzneimittels zur Behandlung eines Patienten mit einer Störung des zentralen Nervensystems.

**21.** Verwendung nach Anspruch 20, wobei die Störung des zentralen Nervensystems ausgewählt ist aus Schlaf/Wach-Störungen, Aufwach/Vigilanz-Störungen, Demenz, Alzheimer-Krankheit, Epilepsie, Narkolepsie, Essstörungen, Bewegungskrankheit, Schwindel, Aufmerksamkeitsstörung mit Hyperaktivität, Lern- und Erinnerungsstörungen, milder kognitiver Beeinträchtigung und Schizophrenie.

**22.** Verwendung nach Anspruch 20, wobei die Störung ausgewählt ist aus Schlaf/Wach-Störungen, Aufwach/Vigilanz-Störungen, Aufmerksamkeitsstörung mit Hyperaktivität und Lern- und Erinnerungsstörungen.

**23.** Verwendung einer Verbindung nach Anspruch 1 zur Herstellung eines Arzneimittels zur Behandlung eines Patienten mit einer allergischen Reaktion der oberen Atemwege.

**24.** Verwendung nach einem der Ansprüche 20 bis 23, wobei die Verbindung eine Formel hat, in der: eines von $R_b$, $R_c$ und $R_e$ WZ ist und die anderen unabhängig ausgewählt sind aus H, Methyl, Ethyl, Methoxy, Ethoxy, Amino und Halo;
$R_a$ und $R_d$ jeweils unabhängig H oder Methyl sind;
W -O- oder $C_{1-3}$-Alkoxy ist;
Z Pyrrolidino, N-Methylpyrrolidino, Pyridyl, Thiazoyl, Piperidino, N-Methylpiperiazino oder $NR_{11}R_{12}$ ist, wobei $R_{11}$ und $R_{12}$ jeweils unabhängig ausgewählt ist aus H, $C_{1-2}$-Alkyl, Phenyl, Benzyl, $C_{3-8}$-Cycloalkyl und einem heterocyclischen $C_{2-5}$-Rest;
und $R_6$ und $R_7$ jeweils unabhängig H, Methyl, Methoxy oder Ethoxy sind.

**25.** Verwendung nach einem der Ansprüche 20 bis 23, wobei die Verbindung eine Formel hat, die ausgewählt ist aus 2-[4-[2-[1-(Methyl)-2-pyrrolidinyl]ethoxy]phenyl)-1-(methylsulfonyl)-1H-indol; 2-[4-[3-Piperidinopropoxy]phenyl)-1-(methylsulfonyl)-1H-indol; 2-[3-[3-Piperidinopropoxy]phenyl)-1-(methylsulfonyl)-1H-indol; und 1-(Methylsulfonyl)-2-(4-(3-(4-methylpiperazino)propoxy)phenyl)indol; oder einem pharmazeutisch verträglichen Salz oder Hydrat davon.

## Revendications

**1.** Composé de la formule (I)(B):

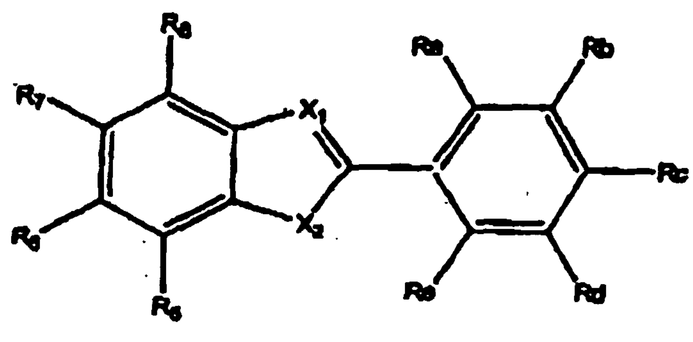

où,

$X_1$ est $CR_1$, où $R_1$ est H, halo, cyano, amino ou nitro;
et $X_2$ est $NR_3$;
$R_3$ est -W'Z';
W' est (C=O) ou $SO_2$;
Z' est alkyle $C_{1-6}$, alcoxy $C_{1-6}$, cycloalkyle $C_{3-8}$, phényl ou un radical hétérocyclique $C_{2-6}$, incluant en option

dans le cycle jusqu'à 3 hétéroatomes ou fragments additionnels indépendamment sélectionnés parmi O, N, NH ,S ,SO et $SO_2$; ou bien Z' est $NR_{13}R_{14}$. où chacun de $R_{13}$ et $R_{14}$ est indépendamment sélectionné parmi alkyle$C_{1-6}$, alkényle$C_{2-6}$, phényle, benzyle, cycloalkyle$C_{3-8}$, et le radical hétérocyclique $C_{2-5}$;
chacun de $R_5$, $R_6$, $R_7$ et $R_8$ est indépendamment H, alkyle $C_{1-6}$, alcoxy$C_{1-6}$, halo, nitro ou amino;
un de $R_a$, $R_b$, $R_c$, $R_d$ et $R_e$ est WZ et les autres sont indépendamment sélectionnés parmi H, alkyle$C_{1-6}$, alcoxy$C_{1-6}$, halo, nitro et amino;
W est -O- ou O-$R_9$, où $R_9$ est alkylène $C_{1-6}$, alkynylène $C_{2-6}$, alkénylène $C_{2-6}$, phénylène ou un radical hétérocyclique bivalent $C_{2-5}$;
Z est un radical hétérocyclique $C_{2-8}$ avec au moins un atome d'azote de base dans le cycle, optionnellement incluant dans le cycle jusqu'à 3 hétéroatomes ou fragments indépendamment sélectionnés parmi O, C=O, N, NH, NG, S, SO et $SO_2$, où G est $R_{15}$, $COR_{15}$, $COOR_{15}$, $SO_2R_{15}$, $SO_2N$, $CSR_{15}$; ou Z est $NR_{11}R_{12}$ où chacun de $R_{11}$ et $R_{12}$ est indépendamment sélectionné parmi H, alkyle$C_{1-6}$, phényle, benzyle, cycloalkyle $C_{3-6}$ et un radical hétérocyclique $C_{2-5}$; ou bien $NR_{11}R_{12}$ pris ensemble est un radical cycloalkylimino $C_{6-8}$; et $R_{15}$ est alkyle$C_{1-6}$, alkynyle$C_{2-6}$, alkényle $C_{2-6}$, cycloalkyle $C_{3-7}$ et cycloalkényle $C_{4-7}$; chacun des groupes hydrocarbyle ou hétérocyclique ci-dessus étant optionnellement substitué par entre 1 et 3 substituants sélectionnés parmi alkyle$C_{1-3}$, alcoxy $C_{1-3}$, halo, hydroxy, phényle et phényl(alkyle$C_{1-3}$); et où chacun des groupes hétérocycliques ci-dessus peut être attaché au reste de la molécule par un atome de carbone ou un hétéroatome; ou un sel pharmaceutiquement acceptable ou hydrate de celui-ci.

**2.** Composé selon la revendication 1, où $R_3$ est - (C=O) alkyle$C_{1-6}$.

**3.** Composé selon la revendication 1, où $R_3$ est -$SO_2$ (alkyle$C_{1-3}$).

**4.** Composé selon la revendication 3, où $R_3$ est méthylsulfonyle.

**5.** Composé selon la revendication 1, où $R_c$ est WZ.

**6.** Composé selon la revendication 1, où $R_b$ ou $R_d$ est WZ.

**7.** Composé selon la revendication 1, où W est éthoxy, propoxy ou butoxy.

**8.** Composé selon la revendication 1, où W est -O-.

**9.** Composé selon la revendication 1, où un de $R_b$, $R_c$ et $R_e$ est WZ et les autres sont indépendamment sélectionnés parmi H, méthyle, éthyle, méthoxy, éthoxy, amino, nitro et halo; et $R_a$ et $R_d$ sont chacun indépendamment H ou méthyle.

**10.** Composé selon la revendication 1, où au moins deux des suivants s'applique: $R_c$ est WZ; W est propoxy ou éthoxy; et Z est N-pipéridino, 2-(N-méthyl)pyrrolidino ou diméthyl amino.

**11.** Composé selon la revendication 1, où Z est pyrrolidino, N-méthyl-pyrrolidino, pyridyle, thiazole, pipéridino, ou $NR_{11}R_{12}$, où chacun de $R_{11}$ et $R_{12}$ est indépendamment sélectionné parmi H, alkyle$C_{1-6}$, phényle, benzyle, cycloalkyle $C_{3-6}$, et un radical hétérocyclique $C_{2-5}$ ou pris ensemble avec N forment un radical cycloalkylamino $C_{6-8}$.

**12.** Composé selon la revendication 1, où un de $R_b$, $R_c$ et $R_e$ est WZ et les autres sont indépendamment sélectionnés parmi H, méthyle, éthyle, méthoxy, éthoxy, amino et halo; et $R_a$ et $R_d$ sont chacun indépendamment H ou méthyle; W est -O- ou alcoxy $C_{1-3}$;
Z est pyrrolidino, N-méthylpyrrolidino, pyridyle, thiazoyle, pipéridino, pipérazino, N-méthylpipérazino ou $NR_{11}R_{12}$, où chacun de $R_{11}$ et $R_{12}$ est indépendamment sélectionné parmi H, alkyle $C_{1-2}$, phényle, benzyle, cycloalkyle$C_{3-6}$ et le radical hétérocyclique $C_{2-5}$; chacun de $R_6$ et $R_7$ sont chacun indépendamment H, méthyle, méthoxy ou éthoxy; chacun de $R_5$ et $R_6$ est H.

**13.** Composé selon la revendication 12, où $R_3$ est -$SO_2$ (alkyle$C_{1-6}$).

**14.** Composé selon la revendication 12, où $R_3$ est $SO_2$(phényle) et (C=O)(alkyle$C_{1-6}$).

**15.** Composé selon la revendication 12, sélectionné parmi 2-[4-[2-[1-(méthyl)-2-pyrrolidinyl] éthoxy] phényl)-1-(méthylsulfonyl)-1H-indole; et 1-(méthylsulfonyl)-2-(4-(3-(4-méthylpipérazino)-propoxy)phényl) indole; ou un sel pharmaceutiquement acceptable ou hydrate de celui-ci.

**16.** Composé selon la revendication 12, sélectionné parmi 2-[4-[3-Pipéridinopropoxy]phényl)-1-(méthylsulfonyl)-1H-indole, et 2-[3-[3-Pipéridinopropoxy]phényl)-1-(méthylsulfonyl)-1H-indole ou un sel pharmaceutiquement acceptable ou hydrate de celui-ci.

**17.** Composition pharmaceutique comprenant un composé de la formule(I)B tel que défini dans la revendication 1 et un support pharmaceutiquement acceptable.

**18.** Composition pharmaceutique selon la revendication 17, où ledit composé a une formule dans laquelle: un de $R_b$, $R_c$ et $R_e$ est WZ et les autres sont indépendamment sélectionnés parmi H, méthyle, éthyle, méthoxy, éthoxy, amino et halo;
Ra et $R_d$ sont chacun indépendamment H ou méthyle;
W est -O- ou alcoxy $C_{1-3}$;
Z est pyrrolidino, N-méthylpyrrolidino, pyridyle, thiazolyle, pipéridino ou $NR_{11}R_{12}$. où chacun de $R_{11}$ et $R_{12}$ est indépendamment sélectionné parmi H, alkyle$C_{1-2}$, phényle, benzyle, cycloalkyle$C_{3-6}$ et le radical hétérocyclique $C_{2-5}$; et
$R_6$ et $R_7$ sont chacun indépendamment H, méthyle, méthoxy ou éthoxy.

**19.** Composition pharmaceutique selon la revendication 21, où ledit composé a une formule sélectionné parmi 2-[4-[2-[1-(méthyl)-2-pyrrolidinyl]éthoxy] phényl)-1-(méthylsulfonyl)-1H-indole; 2-[4-[3-Pipéridinopropoxy]phényl)-1-(méthylsulfonyl)-1H-indole; 2-[3-(3-Pipéridinopropoxy]-phényl)-1-(méthylsulfonyl)-1H-indole; et 1-(méthylsulfonyl)-2-(4-(3-(4-méthylpipérazino)-propoxy)phényl)indole, ou un sel pharmaceutiquement acceptable ou hydrate de celui-ci.

**20.** Utilisation d'un composé selon la revendication 1, dans la fabrication d'un médicament pour traiter un patient ayant un trouble du système nerveux central.

**21.** Utilisation selon la revendication 20, où ledit trouble du système nerveux central est sélectionné parmi des troubles du sommeil/éveil, troubles d'éveil/vigilance, démence, maladie d'Alzheimer, épilepsie, narcolepsie, troubles de l'alimentation, cinétose, vertige, trouble déficitaire de l'attention avec hyperactivité, troubles d'apprentissage et de mémoire, troubles cognitifs légers et schizophrénie.

**22.** Utilisation selon la revendication 20, où ledit trouble est sélectionné parmi des troubles du sommeil/éveil, troubles d'éveil/vigilance, troubles déficitaires de l'attention avec hyperactivité et troubles d'apprentissage et de mémoire.

**23.** Utilisation d'un composé selon la revendication 1 dans la fabrication d'un médicament pour traiter un patient ayant une réponse allergique des voies aériennes supérieures.

**24.** Utilisation selon l'une quelconque des revendications 20 à 23, où ledit composé a une formule dans laquelle: un de $R_b$, $R_c$, et $R_e$ est WZ et les autres sont indépendamment sélectionnés parmi H, méthyle, éthyle, méthoxy, éthoxy, amino et halo;
$R_a$ et $R_d$ sont chacun indépendamment H ou méthyle;
W est -O- ou alcoxy $C_{1-3}$;
Z est pyrrolidino, N-méthylpyrrolidino, pyridyle, thiazolyle, pipéridino, N-méthylpipérazino ou $NR_{11}R_{12}$, où chacun de $R_{11}$ et $R_{12}$ est indépendamment sélectionné parmi H, alkyle$C_{1-2}$, phényle, benzyle, cycloalkyle $C_{3-8}$ et le radical hétérocyclique $C_{2-5}$;
et $R_6$ et $R_7$ sont chacun indépendamment H, méthyle, méthoxy ou éthoxy.

**25.** Utilisation selon l'une quelconque des revendications 20 à 23, où ledit composé a la formule sélectionnée parmi 2-[4-[2-[1-(méthyl)-2-pyrrolidinyl]-éthoxy]phényl)-1-(méthylsulfonyl)-1H-indole; 2-[4-[3-Pipéridinopropoxy]phényl)-1-(méthylsulfonyl)-1H-indole; 2-[3-(3-Pipéridinopropoxy]phényl)-1-(méthylsulfonyl)-1H-indole; et 1-(méthylsulfonyl)-2-(4-(3-(4-méthylpipérazino) propoxy)-phényl)indole; ou un sel pharmaceutiquement acceptable ou hydrate de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5217986 A
- US 5352707 A
- US 5869479 A
- WO 9942458 A
- EP 0978512 A


### Non-patent literature cited in the description


- **PANULA et al.** *Abstr. Society Neuroacience,* 1995, vol. 21, 1977
- **YOKOYAMA et al.** *Eur. J. Pharmacol,* 1993, vol. 234, 129
- **LIN et al.** *Br. Res.,* 1990, vol. 523, 325
- **MONTI et al.** *Eur. J. Pharmacol.,* 1991, vol. 205, 283
- **MACHIDORI et al.** *Brain Research,* 1992, vol. 590, 180
- **BARNES et al.** *Abstr. Society Neuroscience,* 1993, vol. 19, 1813
- **SCHLICKER et al.** *Naunyn Schmiedeberg's Arch. Pharmacol.,* 1996, vol. 353, 326
- **IMAMURA et al.** *J. Pharmacol. Expt. Ther.,* 1994, vol. 271, 1259
- **MCLEOD.** *Abstr. Society Neuroscience,* 1996, vol. 22, 2010
- **ICHINOSE et al.** *Eur. J. Pharmacol.,* vol. 174, 49
- The Histamine H9 Receptor-A Target for New Drugs. Elsevier, 1998
- **GANELLIN et al.** *Arch. Pharm. (Weinhelm, Ger.),* 1998, vol. 331, 395
- **WALCZYNSKI et al.** *Arch. Pharm. (Weinhelm,Ger.),* 1979, vol. 332, 389
- **LINNEY et al.** *J. Med. Chem,* 2000, 2362
- Protective Groups in Organic Synthesis. John Wiley & Sons, 1991
- **R. C. LAROCK.** Comprehensive Organic Transformations. VCH Publishers, 1989, 295-296
- **S.M. BERGE et al.** Pharmaceutical Salts. *J. Pharm. Sci.,* 1977, vol. 66, 1-19
- **GREENE ; WUTS.** Protective Groups in Organic Synthesis. John Wiley & Sons, 1999